# EUROPEAN PATENT APPLICATION

(11) **EP 4 030 169 A1**
(43) Date of publication of application: **20.07.2022**
(21) Application number: 22151534.9
(22) Date of filing: 14.01.2022
(51) Int. Cl.: G01N 35/00, C12Q 1/6844

(54) **NUCLEIC ACID ANALYSIS METHOD AND NUCLEIC ACID ANALYZER**

(30) Priority: 15.01.2021 JP 2021005220
(71) Applicant: Sysmex Corporation, Kobe-shi, Hyogo 651-0073 (JP)
(72) Inventor: Miura, Masahiro, Kobe-shi, Hyogo, 651-0073 (JP); Akama, Kenji, Kobe-shi, Hyogo, 651-0073 (JP); Kawai, Masaaki, Kobe-shi, Hyogo, 651-0073 (JP); Okada, Masaya, Kobe-shi, Hyogo, 651-0073 (JP); Shirai, Kentaro, Kobe-shi, Hyogo, 651-0073 (JP); Yoshida, Yuichiro, Kobe-shi, Hyogo, 651-0073 (JP); Yotoriyama, Tasuku, Kobe-shi, Hyogo, 651-0073 (JP); Notoya, Michitaka, Kobe-shi, Hyogo, 651-0073 (JP); Oue, Soichi, Kobe-shi, Hyogo, 651-0073 (JP)
(74) Representative: Hoffmann Eitle

(57) **Abstract**

PROBLEM TO BE SOLVED: To provide a nucleic acid analysis method capable of reducing reagent cost and improving throughput while guaranteeing the accuracy of nucleic acid analysis. SOLUTION: In a diffusion analysis method, which is an example of an embodiment, a first sample set is prepared that includes a test sample prepared from a first subject sample and a reagent, and at least one control sample prepared from at least one of a positive control and a negative control, and a second sample set is prepared that includes a test sample prepared from a second subject sample and a reagent and does not contain at least one of the control samples contained in the first sample set. The nucleic acid amplification in the first sample set is measured in the first unit, the nucleic acid amplification in the second sample set is measured in the second unit, and the measurement result of each test sample contained in the first sample set and second sample set is analyzed based on the measurement result of the control sample contained in at least the first sample set.

## Description

### CROSS REFERENCE TO RELATED APPLICATIONS

This application claims priority from prior Japanese Patent Application(s) No. 2021-005220, filed on January 15, 2021, entitled "NUCLEIC ACID ANALYSIS METHOD AND NUCLEIC ACID ANALYZER", the entire contents of which are incorporated herein by reference.

### FIELD OF THE INVENTION

The present invention relates to a nucleic acid analysis method and a nucleic acid analyzer.

### BACKGROUND

The global epidemic of COVID-19 has led to a sharp increase in the demand for PCR testing of infectious viruses.

In a general PCR measuring device, amplified nucleic acid is detected by repeating a heating and cooling cycle on a plate having a large number of wells containing a sample. Japanese Patent Publication No. 2008-22732 discloses a method in which a plurality of samples are housed in a plate provided with a plurality of wells, and a large number of samples are nucleic acid-amplified in batch by repeated heating and cooling of the plates.

### PRIOR ART DOCUMENTS

### Patent Documents

Japanese Patent Publication No. 2008-22732

### SUMMARY OF THE INVENTION

### PROBLEM TO BE SOLVED BY THE INVENTION

As disclosed in Japanese Patent Publication No. 2008-22732, in batch processing using a well plate, a standard nucleic acid sample (also referred to as a positive control) and a negative control are included in each batch to guarantee the accuracy of nucleic acid analysis. If a batch always contains a fixed number of positive and negative controls, there is a reagent cost to measure the controls on a batch-by-batch basis. The number of subject samples that can be measured in one batch also is reduced by the number of controls contained in one batch.

The present invention provides a nucleic acid analysis method capable of reducing reagent cost and improving throughput while guaranteeing the accuracy of nucleic acid analysis.

### MEANS FOR SOLVING THE PROBLEM

The present invention provides a nucleic acid analysis method comprising: preparing a first sample set including a test sample prepared from a first subject sample and a reagent, and a control sample prepared from at least one of a positive control and a negative control and the reagent; preparing a second sample set including a test sample prepared from a second subject sample and the reagent, and does not contain at least one of the control samples included in the first sample set; amplifying nucleic acid of the first sample set, and measuring the amplified nucleic acid; amplifying nucleic acid of the second sample set, and measuring the amplified nucleic acid; and analyzing the measurement results of each of the test samples included in the first and second sample sets based on at least the measurement results of the control sample included in the first sample set.

The present invention provides a nucleic acid analyzer comprising: a sample preparation device that prepares a plurality of sample sets including a test sample prepared from a subject sample and a reagent; at least one unit that amplifies nucleic acid of each of the plurality of sample sets and measures the amplified nucleic acid; and a control unit; wherein the sample preparation device, under the control of the control unit, prepares a first sample set including a test sample, a control sample prepared from at least one of a positive control and a negative control, and a second sample set containing a test sample, and does not include at least one of the control samples included in the first sample set; and wherein the control unit analyzes the measurement results of each of the test samples included in the first sample set and second sample set based on at least the measurement results of the control sample included in the first sample set.

### EFFECTS OF THE INVENTION

According to the present invention, it is possible to guarantee the accuracy of nucleic acid analysis while reducing the reagent cost and improving the throughput.

### BRIEF DESCRIPTION OF THE DRAWINGS

FIG. 1 is a schematic view of a nucleic acid analyzer which is an example of an embodiment;
FIG. 2 is a perspective view of a PCR unit;
FIG. 3 is a perspective view of an 8-series tube;
FIG. 4 is a block diagram showing the structure of a nucleic acid analyzer which is an example of an embodiment;
FIG. 5 is a flowchart showing an example of the operation of the nucleic acid analyzer;
FIG. 6 is a flowchart showing an example of the operation of the nucleic acid analyzer;
FIG. 7 is a flowchart showing an example of the operation of the nucleic acid analyzer;
FIG. 8 is a flowchart showing an example of the operation of the nucleic acid analyzer;
FIG. 9 is a flowchart showing an example of an analysis procedure of a measurement result (measurement data);
FIG. 10 is a diagram showing an example of analysis results;
FIG. 11 is a diagram showing an example of sample arrangement in a PCR unit group;
FIG. 12 is a flowchart showing an example of a sample set preparation process;
FIG. 13 shows another example of sample placement in a group of PCR units;
FIG. 14 is a flowchart showing another example of the sample set preparation process;
FIG. 15 shows another example of sample placement in a group of PCR units;
FIG. 16 shows another example of sample placement in a group of PCR units;
FIG. 17 shows another example of sample placement in a group of PCR units;
FIG. 18 is a diagram showing a modified example of the control sample arrangement in a group of PCR units;
FIG. 19 shows another variation of the control sample arrangement in a group of PCR units.
FIG. 20 shows another variation of the control sample arrangement in a group of PCR units.
FIG. 21 is a diagram showing a sample arrangement based on a conventional method;
FIG. 22 is a diagram showing a modified example of a nucleic acid analyzer; and
FIG. 23 is a diagram showing an example of a detection device.

### DESCRIPTION OF THE EMBODIMENTS OF THE INVENTION

Hereinafter, an example of the nucleic acid analysis method and an embodiment of the nucleic acid analyzer according to the present invention will be described in detail with reference to the drawings. The embodiments described below are merely examples, and the present invention is not limited to the following embodiments. It also is within the scope of the present disclosure to selectively combine the components of the plurality of embodiments and modifications described below.

As used herein, the term "sample" refers to a mixture prepared by mixing a sample and a reagent. In the present specification, the term "sample" is is differentiated in meaning according to usage of "test sample" meaning a sample prepared using a subject sample as a sample, and "control sample" prepared using a positive control or a negative control as a sample. "Positive control sample" means a sample prepared using a positive control as a sample. "Negative control sample" means a sample prepared using a negative control as a sample. The "positive control sample" and the "negative control sample" are collectively referred to as "control sample".

In the present specification, a "sample set" is a unit in which a plurality of samples are placed as a set, such that one unit is a number of samples having nucleic acid which can be amplified in one batch process by one unit or less..

As used herein, the term "QC group" means a group of a plurality of samples prepared using one common reagent. The "common reagent" may be a reagent obtained by one preparation, for example, a mixed reagent obtained by mixing a primer reagent and an enzyme reagent. In this case, the mixed reagent contains an amount corresponding to a plurality of tests, and a plurality of samples prepared using the mixed reagent form a single QC group. The "common reagent" also may be a reagent contained in one vial. In this case, a plurality of samples prepared using reagents contained in a common vial form a single QC group. The "common reagent" also may be a reagent of the same production lot. In this case, a plurality of reagents contained in a plurality of different vials and to which the same production lot is given become a common reagent, and a plurality of samples prepared using the reagents of the same production lot form a single QC group.

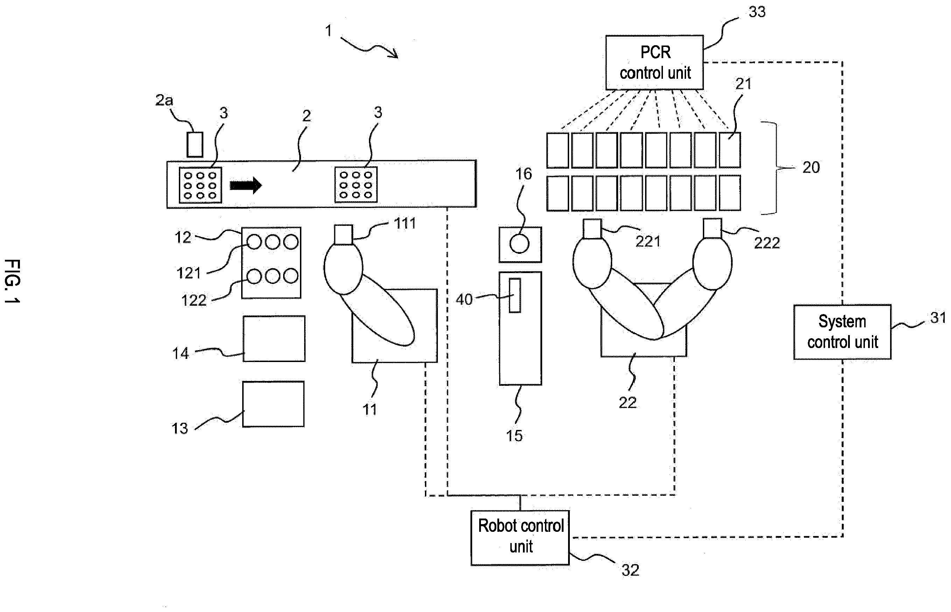

FIG. 1 is a schematic view of a nucleic acid analyzer 1 which is an example of an embodiment. As shown in FIG. 1, the nucleic acid analyzer 1 includes a sample preparation robot 11 that prepares a plurality of sample sets including a test sample prepared from a subject sample and a reagent, PCR unit group 20 for measuring amplified nucleic acid of each sample in a plurality of sample sets, a measurement robot 22 that distributes the sample set prepared by the sample preparation robot 11 to the PCR unit 21 of the PCR unit group 20, a robot control unit 32 that controls each robot, a PCR control unit 33 that controls the PCR unit 21, and a system control unit 31 that controls the overall operation of the nucleic acid analyzer 1 are provided as a main configuration. The PCR unit group 20 includes a plurality of PCR units 21.

The sample preparation robot 11 prepares a first sample set including a test sample and at least one control sample under the control of the robot control unit 32. The sample preparation robot 11 also prepares a second sample set that includes the test sample and does not contain at least one of the control samples included in the first sample set. The control sample included in the first sample set is at least one of a positive control sample prepared from a positive control and a reagent, and a negative control sample prepared from a negative control and a reagent.

The PCR control unit 33 analyzes the measurement result of each test sample included in the first and second sample sets based on the measurement result of the control sample contained in at least the first sample set. By using the measurement results of the control sample contained in the first sample set not only for the analysis of the test sample of the first sample set but also for the analysis of the test sample of the second sample set, the reagent cost can be reduced and the throughput can be improved.

The positive control sample is a sample containing a known concentration of nucleic acid to be tested (also referred to as a target nucleic acid), and is a sample for confirming the accuracy of nucleic acid amplification by the PCR unit 21. Positive control samples are commonly referred to as standard nucleic acid samples, positive controls, quality control samples, or QC samples. The negative control sample is a sample that does not contain at least the nucleic acid of the test control, and is, for example, a sample for confirming the presence or absence of contamination in each process of the nucleic acid analyzer 1. Negative control samples are commonly referred to as negative controls.

The nucleic acid analyzer 1 is, for example, used for a virus test based on a real-time PCR method. The principle of nucleic acid amplification is not limited to the real-time PCR method as long as it amplifies and analyzes nucleic acids, and may be, for example, an isothermal amplification method such as an invader method, a LAMP method, a SmartAmp method, or a TMA method.

An example of a virus to be tested is SARS-CoV-2. The nucleic acid analyzer 1 may form, for example, at least a part of a system that is installed at an airport and performs a virus test fully automatically on a sample collected from a subject who is scheduled to board an airplane.

The subject specimen collected from the subject is, in one example, saliva. The subject specimen may be another type of specimen derived from the respiratory system, such as a pharyngeal swab, a nostril swab, a nasal discharge, sputum. The subject specimen may be whole blood, serum, plasma, cerebrospinal fluid (CSF), pleural effusion, ascites, pericardial fluid, joint fluid, urine, stool, or tissue section.

In the present embodiment, as a subject sample, a nucleic acid extract obtained by extracting nucleic acid contained in saliva collected from a subject is housed in a well plate 3, and supplied to a nucleic acid analyzer 1 by a belt conveyor 2. One or more well plates 3 are sequentially supplied to the belt conveyor 2 in response to the occurrence of an exam request. The well plate 3 has a plurality of wells, and each well individually contains a subject sample, which is a nucleic acid extract. A plurality of subject samples are accommodated in one well plate 3. The nucleic acid analyzer 1 separates a test specimen from the supplied well plate 3, prepares a test sample from the test specimen and the reagent, and performs nucleic acid amplification measurement of each test sample in parallel by a plurality of PCR units 21.

The sample preparation robot 11 prepares an exam sample and a control sample under the control of the robot control unit 32, and prepares a sample set containing a plurality of samples. The sample preparation robot 11 sorts a predetermined amount of the subject sample from the well plate 3 supplied by the belt conveyor 2 and mixes the subject sample and the reagent to prepare a test sample. The sample preparation robot 11 also can prepare a positive control sample and a negative control sample. In the present embodiment, each sample is prepared using an 8-tube unit 40 in which eight containers 41 are connected to prepare a sample set. In other words, a plurality of samples housed in the 8-tube unit 40 is one sample set.

The nucleic acid analyzer 1 includes a belt conveyor 2, a reagent storage unit 12, a QC sample storage unit 13, a nozzle tip storage unit 14, and an 8-series tube storage unit 15 disposed around the sample preparation robot 11.

The belt conveyor 2 is arranged in front of the sample preparation robot 11. The belt conveyor 2 conveys the well plate 3 containing a plurality of nucleic acid extracts as a subject sample to a position accessible to the sample preparation robot 11. The well plate 3 is provided with, for example, 96 wells. The belt conveyor 2 includes a sensor 2a that detects the newly inserted well plate 3. The sensor 2a is, for example, a reflection type optical sensor, and is arranged so as to detect the well plate 3 by irradiating the upper part of the belt conveyor 2 with light and receiving the reflected light. The sensor 2a does not have to be an optical type and may be a contact type. When the sensor 2a is an optical type, the sensor 2a is not limited to the reflective type and may be a transmissive type.

The reagent storage unit 12 includes a freezer for storing the enzyme reagent and a refrigerator for storing the primer reagent. The reagent storage unit 12 stores an enzyme reagent container 121 containing an enzyme reagent and a primer reagent container 122 containing a primer reagent. Enzyme reagents include DNA polymerase, dNTP, and reverse transcriptase as major components. As will be described later, the sample preparation robot 11 separates a predetermined amount of reagents from the enzyme reagent container 121 and the primer reagent container 122, and dispenses the reagents into the mixed reagent container 16 to prepare a reagent used for preparing a sample set. Hereinafter, a reagent prepared by mixing an enzyme reagent and a primer reagent is referred to as a "mixed reagent". The enzyme reagent is preferably stored below freezing (for example, -18 ° C.), and the primer reagent is preferably stored at a low temperature of 1°C to 5°C.

The QC sample storage unit 13 includes, for example, a refrigerator for storing positive and negative controls at a low temperature of 1°C to 5°C. A positive control is a sample containing a known concentration of target nucleic acid or a buffer containing a known concentration of target nucleic acid. The target nucleic acid is, for example, the nucleic acid (RNA or artificially synthesized DNA) or nucleic acid fragment of the SARS-CoV-2 virus when the test target is the SARS-CoV-2 virus. Negative controls are samples that do not contain the target nucleic acid, such as pure water or buffer.

The nozzle tip storage unit 14 stores a plurality of nozzle tips that can be attached to and detached from the tip of the robot arm by the sample preparation robot 11. The nozzle tip is made of a disposable material and is discarded after use by a sample preparation robot.

The 8-series tube storage area 15 is arranged between the sample preparation robot 11 and the measurement robot 22, and holds a plurality of 8-series tubes 40.

The sample preparation robot 11 is, for example, a vertical articulated robot. The sample preparation robot 11 includes an end effector 111 at the tip of the arm. The end effector 111 is provided with a syringe pump as a tool, and the nozzle tip in the nozzle tip storage unit 14 can be detachably attached to the tip.

When preparing the mixed reagent, the sample preparation robot 11 uses the nozzle tip attached to the end effector 111 to suction predetermined amounts of the enzyme reagent in the enzyme reagent container 121 stored in the reagent storage unit 12 and the primer in the primer reagent container 122, and dispense the suctioned reagents into the mixed reagent container 16.

When the prepared mixing reagent is dispensed, the sample preparation robot 11 suctions a predetermined amount of the mixed reagent contained in the mixing reagent container 16 using the nozzle tip attached to the end effector 111, and dispenses a predetermined amount of the mixed reagent into the container 41 of the 8-tube unit 40.

When the sample preparation robot 11 dispenses the subject sample, the subject sample accommodated in the well of the well plate 3 transported by the belt conveyor 2 is suctioned using the nozzle tip attached to the end effector 111, and dispensed into the container 41 of the 8-tube unit 40.

When the positive control or the negative control is dispensed, the sample preparation robot 11 suctions a predetermined amount of the positive control and the negative control of the QC sample storage unit 13 using the nozzle tip attached to the end effector 111, and dispenses the positive and negative controls to the container 40 of the 8-tube unit 40.

The sample preparation robot 11 prepares a test sample by dispensing and mixing the subject sample and the mixing reagent into the container 41 of the 8-tube unit 40. Similarly, the sample preparation robot 11 prepares a control sample by dispensing and mixing a positive control or a negative control and a mixed reagent into a container 41 of an 8-tube unit 40. Since eight containers 41 are present in one 8-tube unit 40, a sample set containing up to eight samples can be prepared. If the sample set includes two types of control samples, the maximum number of test samples is six. Although details will be described later, in the present embodiment, a predetermined amount (sufficient mixed reagent for 48 tests) is prepared from a predetermined amount of the enzyme reagent suctioned from one enzyme reagent container 121 and a predetermined amount of the primer reagent suctioned from one primer reagent container 122. A positive control sample and a negative control sample are prepared one by one each time a mixed reagent is prepared. For example, when 48 tests of mixed reagent are prepared, the sample set initially prepared contains one positive control sample, one negative control sample, and six test samples.

The PCR unit group 20 is configured by a plurality of PCR units 21 capable of amplifying nucleic acids and measuring amplified nucleic acids independently and in parallel with each other. The PCR unit 21 is a measurement unit that amplifies the nucleic acid contained in the sample set and measures the amplified nucleic acid under the control of the PCR control unit 33. In the present embodiment, the measuring robot 22 sequentially distributes each sample set (8-tube 40) to a plurality of PCR units 21 according to a predetermined rule. One 8-tube unit 40 can be set in one PCR unit 21. A maximum of eight samples can be simultaneously subjected to nucleic acid amplification processing in one PCR unit 21.

In the present embodiment, the PCR unit group 20 is composed of 16 PCR units 21 (hereinafter, may be referred to as "PCR units U1 to U16"). Each PCR unit 21 is a measurement unit of the same type, and mutually perform the same processing and measurement.

The measuring robot 22 is a dual-arm robot and includes two robot arms. End effector 221 and end effector 222 are provided at the tips of the two robot arms, respectively. A robot hand is attached to the end effectors 221 and 222 as a tool capable of grasping and moving the 8-tube unit 40. Under the control of the robot control unit 32, the measuring robot 22 sets the 8-tube unit 40 containing the sample set in the PCR unit 21. The measuring robot 22 takes out the 8-tube unit 40 for which the measurement has been completed from the PCR unit 21 and discards it.

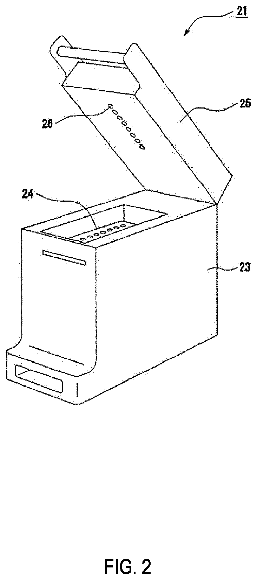

FIG. 2 is a perspective view of the PCR unit 21. The PCR unit 21 has a thermal cycler 23, a tube holding portion 24 in which the 8-tube unit 40 is set, and an openable/closable cover 25 on which the optical portion 26 is mounted. The PCR unit 21 performs reverse transcription process and nucleic acid amplification process on the test sample and the control sample, and measures the nucleic acid amplification of each sample. The thermal cycler 23 is a device that heats and cools the 8-tube unit 40 set in the tube holding portion 24. The reverse transcription process of the PCR unit 21 is performed by heating the sample to a predetermined temperature (for example, 45°C) for a certain period of time. By heating the sample, the reverse transcriptase contained in the enzyme sample promotes the reverse transcription reaction of the nucleic acid in the sample.

The PCR unit 21 starts the nucleic acid amplification process after the reverse transcription process is completed. The nucleic acid amplification process is a process of repeating a nucleic acid amplification cycle in which a sample is heated and cooled at a predetermined cycle. In the nucleic acid amplification process, for example, a nucleic acid amplification cycle in which heating to 95°C and cooling to 60°C are repeated at a cycle of 75 seconds is performed 45 times. A Perche element is mounted on the thermal cycler 23, and the nucleic acid amplification process of each sample is performed by heating and cooling the 8-tube unit 40 in which the Perche element is set in the tube holding portion 24. The detection of the nucleic acid amplified by this process is performed by the optical unit 26 mounted on the cover 25.

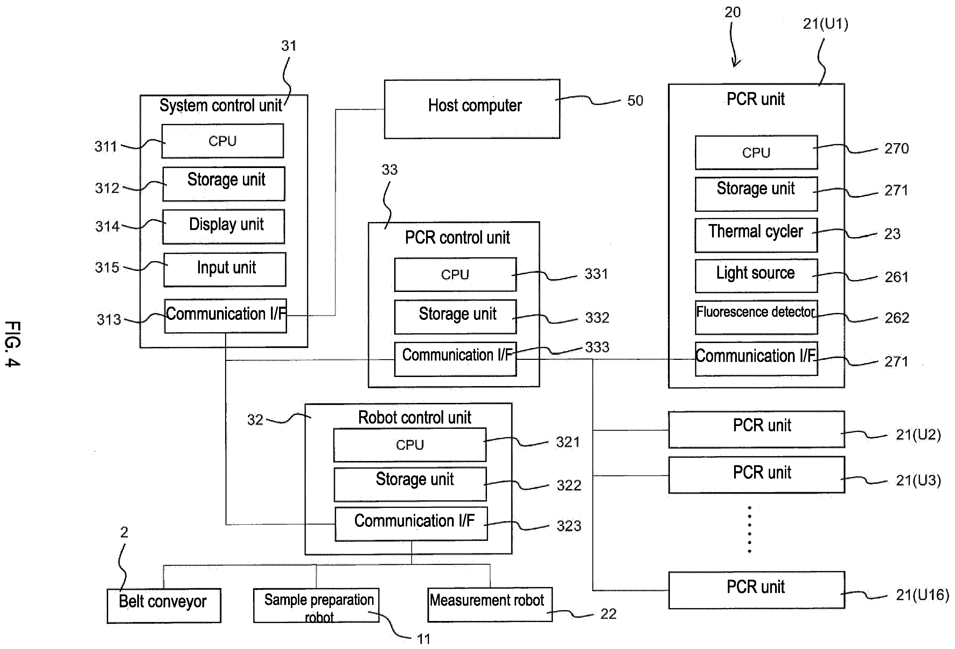

The optical unit 26 includes a light source 261 and a fluorescence detector 262 (see FIG. 4). The light source 261 is provided so as to face the 8-tube unit 40 set in the tube holding portion 24 with the cover 25 closed, and irradiates excitation light on the sample contained in each container 41. The fluorescence detector 262 detects the fluorescence of the sample generated by the light irradiation of the light source 261 with the cover 25 closed. An example of the light source 261 is an LED, and an example of the fluorescence detector 262 is a photodiode. On the cover 25, for example, a plurality of light sources 261 and a fluorescence detector 262 are installed in a line along the direction in which the containers 41 of the 8-tube tube 40. Note that the method for introducing the fluorescent substance used for the PCR measurement into the sample is not particularly limited, and may be any of, for example, an intercalator method, a probe method, a cycling probe method, and a calcein method. Alternatively, a method of measuring turbidity or absorbance without using a fluorescent substance also may be used.

In PCR measurement, when nucleic acid is amplified by repeating the nucleic acid amplification cycle, the fluorescence intensity detected by the fluorescence detector 262 increases. The number of cycles in which the fluorescence intensity exceeds a predetermined threshold is faster when the sample contains a large amount of the target nucleic acid to be tested, and slower when the sample contains no or a small amount of the target nucleic acid. Alternatively, when the sample does not contain the target nucleic acid, the fluorescence intensity does not change even if the number of cycles increases. Therefore, the presence or absence of the target nucleic acid in the sample can be confirmed by using this number of cycles. The PCR control unit 33 acquires, for example, the fluorescence intensity detected by the fluorescence detector 262, and determines the number of cycles when the fluorescence intensity exceeds a predetermined threshold value. The number of cycles when the fluorescence intensity exceeds a predetermined threshold value is called a Ct value.

The PCR control unit 33 creates, for example, an amplification curve of fluorescence intensity or an amplification curve of nucleic acid based on fluorescence intensity, compares a predetermined threshold value set for the fluorescence intensity with the amplification curve, and calculates the number of nucleic acid amplification cycles when the fluorescence intensity amount exceeds the threshold value as a Ct value. The calculation of the Ct value is not limited to the method of comparing the fluorescence intensity and the threshold value, and the quadratic differential curve of the amplification curve may be obtained and the point where the value becomes the maximum may be used as the Ct value. If the Ct value is less than the predetermined number of cycles, the sample is likely to be positive, and if the Ct value is greater than or equal to the predetermined number of cycles, the sample is likely to be negative. The PCR control unit 33 stores the Ct value in association with the information that identifies each sample. The PCR control unit 33 also may store the amplification curve together with the Ct value. The identification information of the test sample is, for example, a sample number assigned to each subject.

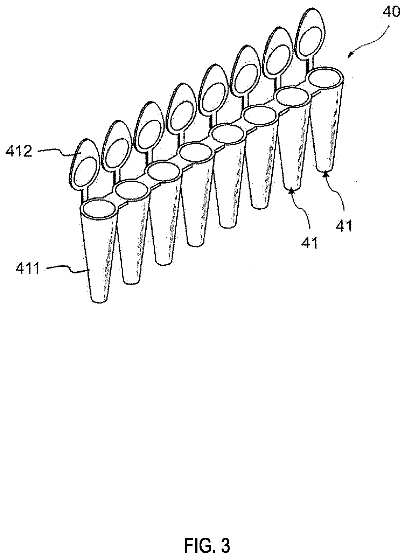

FIG. 3 is a perspective view of the 8-tube unit 40. As shown in FIG. 3, the 8-tube unit 40 is a group of containers in which eight containers 41 are arranged in a row and adjacent containers 41 are connected to each other and integrated. Since the 8-tube unit 40 has a structure in which eight independent containers 41 are integrated, eight containers 41 containing eight samples can be simultaneously conveyed, which contributes to an improvement in throughput. Each container 41 has a bottomed cylindrical container body 411 in which a sample is stored, and a lid 412 that closes the opening of the container body 411. The container body 411 and the lid 412 are made of, for example, a translucent resin.

The sample set housed in one 8-tube unit 40 is made using the same mixing reagents dispensed from the same mixing reagent container 16. In this embodiment, a plurality of sample sets (for example, 6 sample sets) housed in a plurality of 8-series tubes 40 are prepared using the same mixed reagent. As will be described in detail later, in one mixed reagent container 16, an amount of mixed reagents capable of preparing 48 test samples constituting the six sample sets is prepared, and the QC group includes at least one positive control sample and one negative control sample.

FIG. 4 is a block diagram showing the structure of the nucleic acid analyzer 1. As shown in FIG. 4, the belt conveyor 2, the sample preparation robot 11, and the measurement robot 22 are communicably connected to the robot control unit 32, and the operations thereof are controlled by the robot control unit 32. Each PCR unit 21 configuring the PCR unit group 20 is communicably connected to the PCR control unit 33, and the operations thereof are controlled by the PCR control unit 33. The PCR control unit 33 acquires the fluorescence intensity detected by the fluorescence detector 262 from each PCR unit 21, creates an amplification curve of the fluorescence intensity, and obtains a Ct value. The robot control unit 32 and the PCR control unit 33 are communicably connected to the system control unit 31.

The system control unit 31 is a device that comprehensively controls the nucleic acid analyzer 1, and includes a CPU 311, a storage unit 312, a communication interface (IF) 313, a display unit 314, and an input unit 315. The system control unit 31 may be configured by a personal computer. The system control unit 31 comprehensively controls the operation of the nucleic acid analyzer 1 by executing the software stored in the storage unit 312, and executes processes to output a control command to the robot control unit 32 and the PCR control unit 33, creates a sample set, distribute the sample set, perform PCR measurement, and analyze the measurement result (measurement data). The system control unit 31 is communicably connected to the host computer 50, and transmits the analysis result analyzed by the PCR control unit 33 to the host computer 50. The host computer 50 is used, for example, when a doctor confirms the analysis result and makes a positive/negative determination for each test sample.

The storage unit 312 is configured by a RAM, a ROM, a hard disk, and the like. A computer program for executing the above processing is stored in the storage unit 312. This computer program is executed by the CPU 311. The display unit 314 is composed of a display and displays the operating status of the nucleic acid analyzer 1, the analysis result, and the like. The input unit 315 is is configured by a keyboard and a mouse, and is used, for example, for inputting identification information of a sample housed in a well plate 3 and input of a sample test request. The communication interface 313 includes, for example, an Ethernet-compatible communication module.

The robot control unit 32 is a device that controls the belt conveyor 2, the sample preparation robot 11, and the measurement robot 22, and includes a CPU 321, a storage unit 322, and a communication interface 323. The robot control unit 32 controls the operations of the belt conveyor 2, the sample preparation robot 11, and the measurement robot 22 based on the control command of the system control unit 31.

Similar to the robot control unit 32, the PCR control unit 33 has a CPU 331, a storage unit 332, and a communication interface 333, and controls the operation of each PCR unit configuring the PCR unit group 20 based on the control command of the system control unit 31. The PCR control unit 33 may be configured by a personal computer.

The PCR control unit 33 acquires information about each sample to be distributed to each PCR unit 21 from the system control unit 31 and stores the information in the storage unit 332. The information related to each sample is the identification information of each sample and is associated with the position information of the container 41 of the 8-tube unit 40 containing the sample. The identification information of each sample is, for example, the sample number if the sample is a test sample, and the information indicating the type of the sample or the lot number of the control sample if the sample is a control sample. As described above, the PCR control unit 33 acquires measurement data (fluorescence intensity detected by the fluorescence detector 262) from each PCR unit 21, creates an amplification curve, and calculates a Ct value. The PCR control unit 33 outputs the analysis result in which the identification information of each sample is associated with the amplification curve and the Ct value to the system control unit 31.

The PCR unit 21 includes a thermal cycler 23 including a Perche element, a light source 261 and a fluorescence detector 262 mounted on a cover 25, a CPU 270, a storage unit 271, and a communication interface 271. The CPU 270 controls the operations of the thermal cycler 23, the light source 261 and the like by executing the program stored in the storage unit 271 in response to the control command transmitted from the PCR control unit 33.

Under the control of the PCR control unit 33 and the CPU 270, the thermal cycler 23 heats and cools the 8-tube unit 40 set in the tube holding unit 24 to perform reverse transcription processing and nucleic acid amplification processing. The light source 261 irradiates each container 41 of the 8-tube unit 40 with excitation light under the control of the PCR control unit 33 and the control unit 27. Under the control of the PCR control unit 33 and the control unit 27, the fluorescence detector 262 detects the fluorescence intensity generated from the sample in each cycle of nucleic acid amplification, and sends measurement data consisting of time-series data of the fluorescence intensity to the PCR control unit 33.

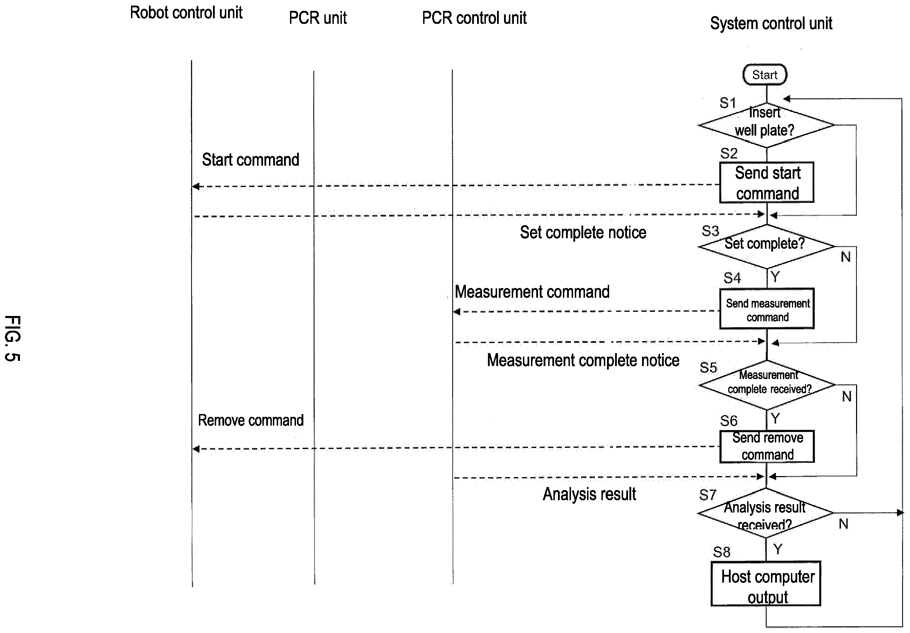

Hereinafter, an example of the analysis process by the nucleic acid analyzer 1 will be described in detail with reference to FIGS. 5 to 12. FIGS. 5 to 8 are flowcharts showing the operation of the nucleic acid analyzer 1, wherein the four control bodies (system control unit 31, robot control unit 32, PCR control unit 33, and PCR unit 21) configuring the nucleic acid analyzer 1 are shown.

FIG. 5 is a flowchart showing the operation of the system control unit 31. In step S1, the system control unit 31 determines whether a new well plate 3 has been put into the belt conveyor 2. The determination in step S1 is determined YES based on the sensor 2a detecting a new well plate 3 installed on the belt conveyor 2 and the detection signal from the sensor 2a being input to the system control unit 31 via the robot control unit 32. If YES is determined in step S1, the process proceeds to step S2. Note that the determination in step S1 may be determined to be YES when the user inputs an inspection request via the input unit 315 instead of the detection by the sensor 2a.

In step S2, the system control unit 31 sends a start command to the robot control unit 32 to start a series of analysis processes including the preparation of the sample set, the PCR measurement, and the analysis of the measurement data. As will be described in detail later, the robot control unit 32 that has received the start command controls the sample preparation robot 11 to prepare a sample set from the sample of the subject of the well plate 3, and the 8-tube unit 40 containing the sample set. Is set in any PCR unit 21 determined according to a predetermined rule.

In step S3, the system control unit 31 determines whether the set completion notification has been received from the robot control unit 32. As will be described later, the set completion notification is a notification that the 8-tube unite 40 has been set in the PCR unit 21, and includes identification information U1 to U16 of one PCR unit 21 in which the 8-tube unit 40 is set. When the set completion notification is received, the system control unit 31 transmits a measurement command to the PCR control unit 33 (step S4). The measurement command is a control command for starting PCR measurement in the PCR unit 21 corresponding to the set completion notification. As will be described in detail later, the PCR control unit 33 that has received the measurement command sends a control command to the designated PCR unit 21 to start the PCR measurement, and when the measurement is completed, sends a measurement completion notification to the system control unit 31.

In step S5, the system control unit 31 determines whether the measurement completion notification has been received from the PCR control unit 33. When receiving the measurement completion notification, the system control unit 31 transmits a take-out command for removing the 8-tube unit 40 from the PCR unit 21 to the robot control unit 32 (step S6).

In step S7, the system control unit 31 determines whether the analysis result has been received from the PCR control unit 33. When the analysis result is received, the system control unit 31 outputs the analysis result to the host computer 50 in step S8. Note that the order of steps S6 and steps S7 and S8 also may be reversed. When there are a plurality of host computers 50, the system control unit 31 may select the host computer 50 to which the analysis result is transmitted based on the identification information of the sample. The analysis result received from the PCR control unit 33 plus additional information also may be output to a predetermined host computer 50. When the system control unit 31 outputs the analysis result, the system control unit 31 returns the process to step S1.

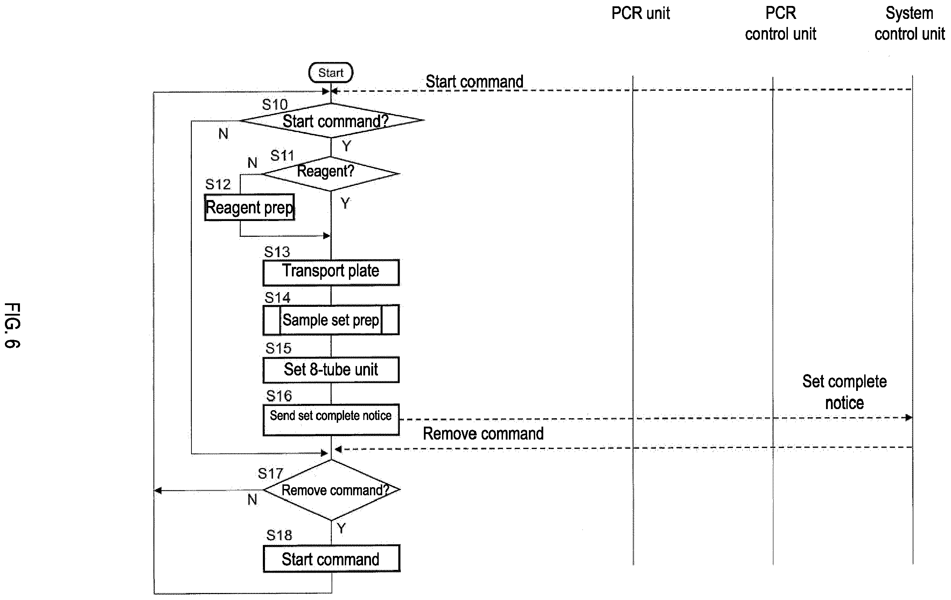

FIG. 6 is a flowchart showing the operation of the robot control unit 32. In step S10, the robot control unit 32 determines whether a start command has been received from the system control unit 31. When the start command is received, the robot control unit 32 confirms the presence or absence of the mixing reagent necessary for preparing the sample set (step S11). If there is a mixed reagent, the process proceeds to step S13. When there is no mixed reagent, that is, when the number of remaining tests of the prepared mixed reagent is zero, the robot control unit 32 controls the sample preparation robot 11 to prepare the mixed reagent (step S12). Under the control of the robot control unit 32, the sample preparation robot 11 suctions a predetermined amount of reagents from the enzyme reagent container 121 and the primer reagent container 122 in the reagent storage unit 12, dispenses the reagents into the mixing reagent container 16 to prepare the mixing reagent. In this embodiment, sufficient mixed reagent for 48 tests is prepared in one preparation of mixed reagents.

In step S13, the robot control unit 32 controls the belt conveyor 2 to move the well plate 3 to a position accessible by the sample preparation robot 11. In step S14, the robot control unit 32 controls the sample preparation robot 11 to prepare a test sample from the subject sample contained in each well of the well plate 3 and the mixed reagent contained in the mixed reagent container 16 to produce a sample set. Under the control of the robot control unit 32, the sample preparation robot 11 prepares, for example, a sample set containing only the test sample and a sample set containing the test sample and the control sample. In step S14, the sample preparation robot 11 dispenses the subject sample, the mixed reagent, and the like into each container 41 of the 8-tube unit 40 in the 8-tube unit storage area 15 to prepare a sample and produce a sample set containing a maximum of 8 samples. The process of step S14 will be described later.

In step S15, the robot control unit 32 determines which of the PCR units 21 of U1 to U16 the 8-tube unit 40 is to be set according to a predetermined rule. The robot control unit 32 controls the measurement robot 22 to open the cover 25 of the PCR unit 21 determined as the transfer destination, sets the 8-tube unit 40 in the tube holding unit 24, and closes the cover 25. The predetermined rule is, for example, U1, U2, U3 ... U16 in that order. When the 8-tube unit 40 is set in the tube holding unit 24, for example, the sensor of the PCR unit 21 detects the 8-tube unit 40, and the detection signal of the sensor is transmitted to the robot control unit 32 and the PCR control unit 33. In step S16, the robot control unit 32 transmits a set completion notification to the system control unit 31. The robot control unit 32 transmits, for example, a set completion notification when the detection signal is received. As described above, the set completion notification includes identification information of the PCR unit in which the 8-tube unit 40 is set, for example, any one of U1 to U16.

When the 8-tube unit 40 is set in the PCR unit 21, PCR measurement is started by the PCR unit 21 under the control of the system control unit 31 and the PCR control unit 33.

The robot control unit 32 determines whether the PCR measurement for each sample of the 8-tube unit 40 is completed and the take-out command is received from the system control unit 31 (step S17). When the take-out command is received, the robot control unit 32 controls the measurement robot 22 to remove the measured 8-tube unit 40 from the PCR unit 21 (step S18). Specifically, the measuring robot 22 takes out the 8-tube unit 40 corresponding to the take-out command from the PCR unit 21 and moves it to a predetermined place under the control of the robot control unit 32. The predetermined location is, for example, a disposal box containing the measured 8-tube unit 40.

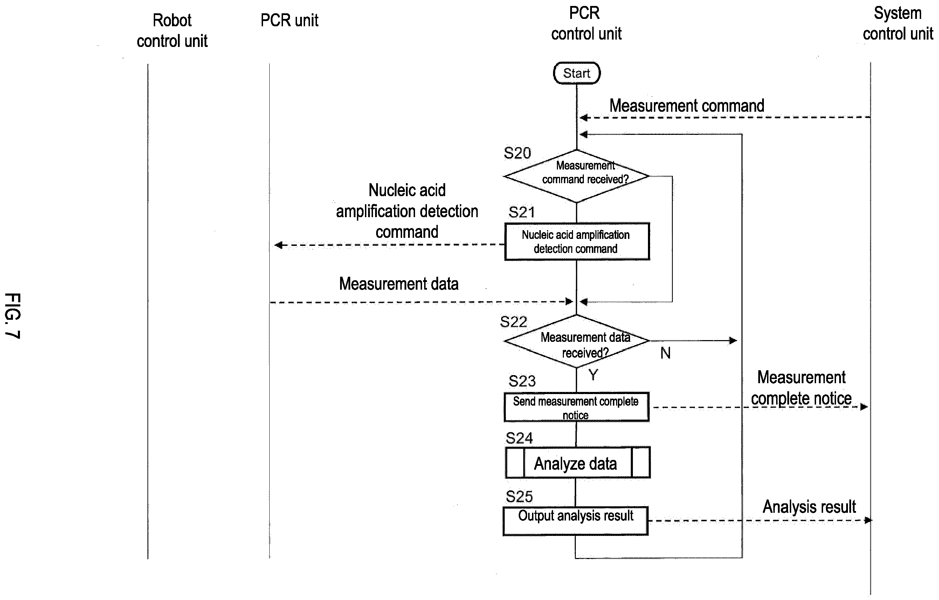

FIG. 7 is a flowchart showing the operation of the PCR control unit 33. In step S20, the PCR control unit 33 determines whether a measurement command has been received from the system control unit 31. When the measurement command is received, the PCR control unit 33 transmits a nucleic acid amplification/detection command to the designated PCR unit 21 corresponding to the measurement command (step S21). As will be described in detail later, the PCR unit 21 that has received the nucleic acid amplification/detection command performs PCR measurement on each sample of the 8-tube units 40 set in the tube holding unit 24, and transmits the measurement data to the PCR control unit 33. Each sample of the 8-tube unit 40 is simultaneously subjected to nucleic acid amplification processing and irradiated with excitation light, and fluorescence generated from each sample is detected.

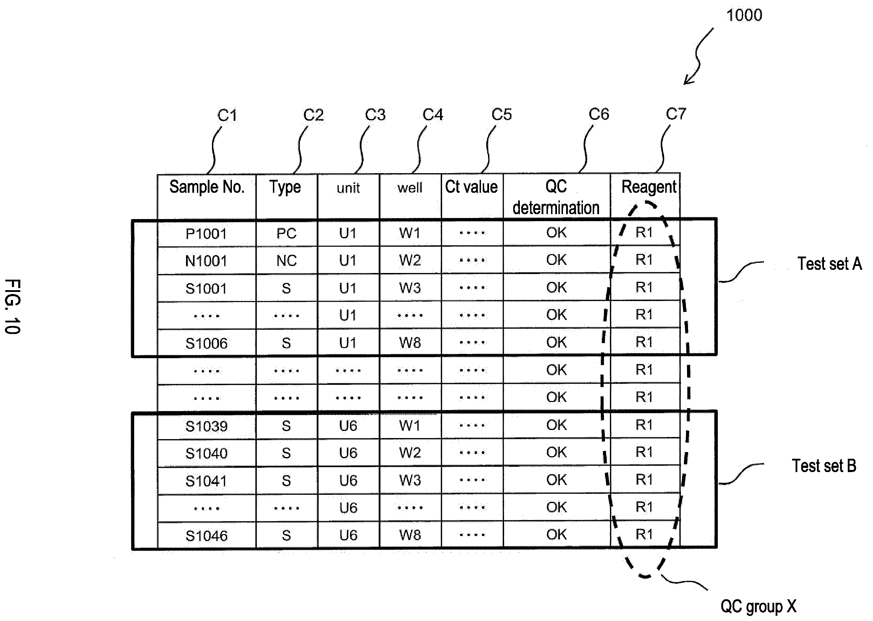

The measurement command includes identification information of the target PCR unit and identification information of each sample of the 8-tube unit 40 (sample set) set in the tube holding unit 24. The identification information of each sample is associated with the number of the container 41 of the 8-tube unit 40, is stored in the storage unit 332, and is used when analyzing the measurement data (see FIG. 10 described later). That is, the PCR control unit 33 has information on what kind of sample is contained in which container 41 of the 8-tube unit 40 set in each PCR unit 21 based on the measurement command. In this way the PCR control unit 33 can output an analysis result in which the amplification curve and Ct value obtained from the measurement result of each sample in each PCR unit 21 and the identification information of each sample are associated with each other.

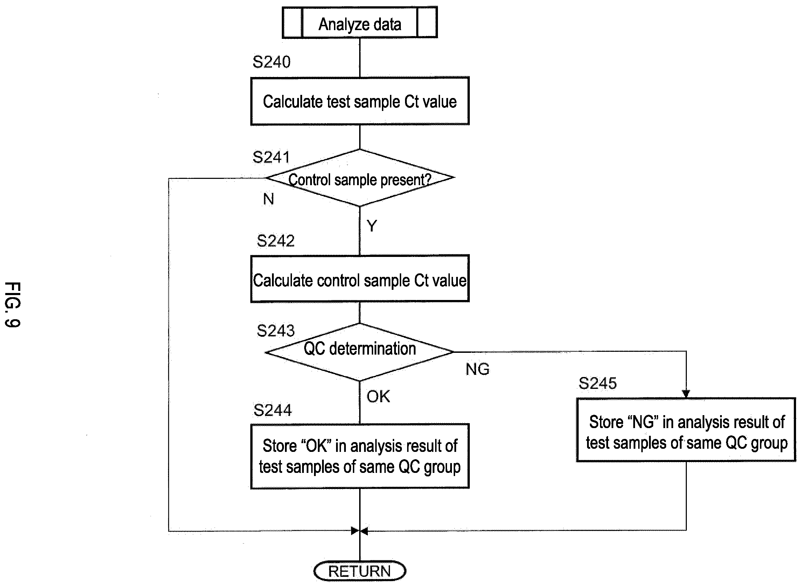

In step S22, the PCR control unit 33 determines whether the measurement data have been received from the PCR unit 21. When the measurement data are received, the PCR control unit 33 transmits a measurement completion notification to the system control unit 31 (step S23). As described above, the system control unit 31 that has received the measurement completion notification transmits a removal command to the robot control unit 32 (step S6 in FIG. 5). The PCR control unit 33 analyzes the measurement data received in step S24 to create an analysis result, and transmits the analysis result to the system control unit 31 in step S25. The measurement data are analyzed by the procedure shown in FIG. 9 described later. The analysis result is transmitted to the host computer 50 via the system control unit 31 (step S8 in FIG. 5), and is used for a positive/negative determination by a doctor.

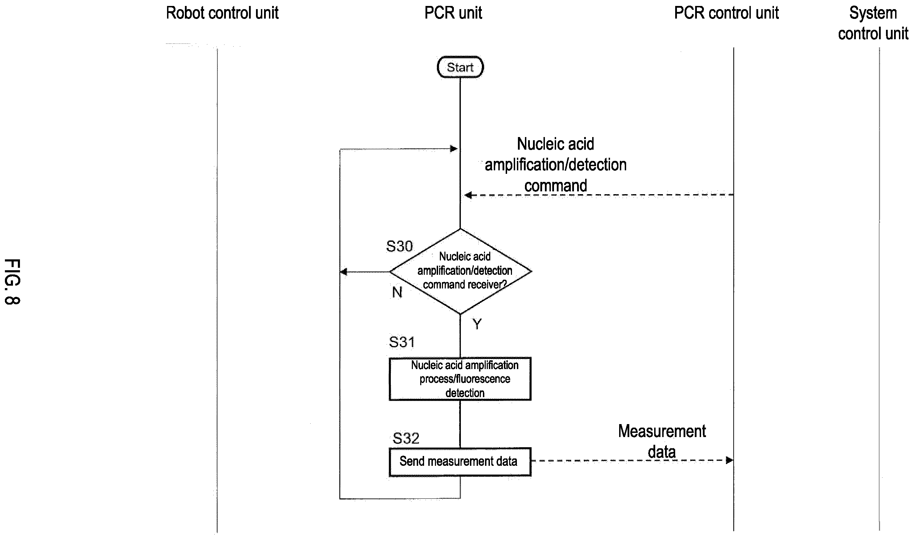

FIG. 8 is a flowchart showing the operation of the PCR unit 21. In step S30, the PCR unit 21 determines whether a nucleic acid amplification/detection command has been received from the PCR control unit 33. Upon receiving the nucleic acid amplification/detection command, the PCR unit 21 performs nucleic acid amplification processing and fluorescence detection in step S31. Specifically, the PCR unit 21 heats the 8-tube unit 40 with the thermal cycler 23 to perform reverse transcription processing, and then repeats a nucleic acid amplification cycle of heating and cooling the 8-tube unit 40 a predetermined number of cycles to perform the nucleic acid amplification process. In this embodiment, the nucleic acid amplification cycle of heating to 95°C and cooling to 60°C is performed 45 times. In each cycle of nucleic acid amplification, the PCR unit 21 irradiates each container 41 of the 8-tube units 40 with excitation light by the light source 261 and detects the fluorescence generated from the sample by the fluorescence detector 262.

Fluorescence detection is performed at a specific timing in the nucleic acid amplification cycle. For example, when the sample is cooled to 60°C, the intensity of fluorescence generated from the sample is measured by irradiating with excitation light. The PCR unit 21 measures the fluorescence intensity in each cycle of nucleic acid amplification for all the samples contained in the 8-tube unit 40. In step S32, the PCR unit 21 transmits this measurement data to the PCR control unit 33. The measurement data may be transmitted every time the nucleic acid amplification cycle is completed, or may be transmitted collectively after all the nucleic acid amplification cycles are completed and 45 measurements are performed.

FIG. 9 is a flowchart showing a procedure for analyzing measurement data in the PCR control unit 33.

In step S240, the PCR control unit 33 obtains the Ct value of the test sample based on the measurement data of the test sample among the measurement data corresponding to one sample set received in step S22. Specifically, the PCR control unit 33 creates, for example, an amplification curve of the fluorescence intensity based on the measurement data, and calculates the Ct value based on the number of cycles in which the fluorescence intensity exceeds the threshold value. The amplification curve and Ct value are stored in the storage unit 332 of the PCR control unit 33. The threshold value is set, for example, to a signal level at which a significant increase is seen relative to the baseline signal of the measured fluorescence intensity, and is stored in advance in the storage unit 332.

In step S241, the PCR control unit 33 determines whether the received measurement data includes the measurement data of the control sample. If the measurement data of the control sample are not included, the process of FIG. 9 ends and returns to the main routine. When the measurement data of the control sample are included, the PCR control unit 33 obtains the Ct value of the control sample based on the measurement data of the control sample in the same manner as the process described in step S240 (step S242).

In step S243, the PCR control unit 33 makes an accuracy control determination based on the Ct value of the control sample calculated in step S242.

The quality control determination for the positive control sample is performed by confirming whether the Ct value of the positive control sample is equal to or less than the first cycle number (for example, 32 cycles) set as the threshold value. When the Ct value is equal to or less than the number of first cycles, it is determined that there is no abnormality. When the Ct value exceeds the number of first cycles, it is determined that there is an abnormality. The first number of cycles is the number of cycles that the positive control sample should show at least when there is no abnormality in the series of processes including the quality of the reagent, the sample preparation step, and the nucleic acid amplification step, and is stored in advance in the storage unit 332 of the PCR control unit 33. If the Ct value of the positive control sample exceeds the number of the first cycle, it means that the rise of fluorescence intensity is slower than the standard, that the reagent of the desired quality has not been prepared, or that the nucleic acid amplification cycle is not functioning normally, such that an abnormality is suspected.

For the negative control sample, the quality control determination is performed in the same manner as for the positive control sample. The quality control determination for the negative control sample is performed by confirming whether the Ct value of the negative control sample is equal to or greater than the second cycle number (for example, 40 cycles) set as the threshold value. When the Ct value is equal to or greater than the second number of cycles, it is determined that there is no abnormality. If the Ct value is smaller than the number of second cycles, it is determined that there is an abnormality. The second number of cycles is the number of cycles that the negative control sample does not show when there is no abnormality in the process series, and is stored in advance in the storage unit 332 of the PCR control unit 33. If the Ct value of the negative control sample is smaller than the number of second cycles, it means that the fluorescence intensity rises faster than the standard, and it is possible that the negative control sample contains the target nucleic acid that should not have been originally contained. In this case, the occurrence of contamination is suspected.

When the quality control determination of the positive control sample and the negative control sample is "no abnormality", the PCR control unit 33 stores the "OK" flag with the analysis result of the same QC group prepared using the same mixed reagent as the control sample (step S244). On the other hand, when the quality control determination of either the positive control sample and the negative control sample is "abnormal", the PCR control unit 33 stores the "NG" flag with the analysis result of the same QC group prepared using the same mixed reagent as the control sample (step S245).

FIG. 10 is a schematic diagram of an analysis result database 1000 (hereinafter, simply referred to as DB) stored in the storage unit 332 of the PCR control unit 33. The DB 1000 is created in the storage unit 332 of the PCR control unit 33. The DB1000 includes a column C1 in which the sample number is stored, a column C2 in which the sample type is stored, a column C3 in which the identification information of the PCR unit used for nucleic acid analysis is stored, a column C4 in which the well number accommodating the sample is stored, a column C5 in which the Ct value is stored, a column C6 in which the determination result of quality control is stored, and a column C7 in which the identification information of the used reagent is stored.

The information in columns C1 to C4 is included in the measurement command received by the PCR control unit 33 from the system control unit 31 in step S20. As the Ct value in column C5, the Ct value of the test sample obtained in step S240 is stored in the record of the test sample. For the control sample record, the Ct value of the control sample obtained in step S242 is stored. In column C6, either the "OK" or "NG" flag is stored as a result of the PCR control unit 33 determining based on the Ct value of the control sample in step S243.

In the example of FIG. 10, the sample set A contains a positive control sample P1001, a negative control sample N1001, and six test samples S1001 to S1006. The sample set B contains eight test samples S1039 to S1046. Sample set A and sample set B configure QC group X prepared based on the same mixed reagent R1. In the example of FIG. 10, the quality control determination of the positive control sample P1001 and the negative control sample N1001 included in the sample set A is OK. In this case, "OK" is stored as the quality control determination result of the six test samples included in the same sample set A as the positive control test P1001 and the negative control sample N1001. "OK" is also stored in the quality control determination result of the inspection samples S1039 to 1046 of the sample set B included in the same QC group X. Although not shown, "OK" is also stored in the result of the quality control determination of the test sample included in the sample sets other than the sample sets A and B included in the same QC group X.

When the quality control determination of both or either of the positive control sample P1001 and the negative control sample N1001 is "abnormal", "NG" is stored in the quality control determination result of all the samples included in the same QC group X including the test samples of the sample sets A and B, that is, the test samples S1001 to S1046 (step S245). When the quality control determination result is NG, and the analysis result is output from the PCR control unit 33 to the system control unit 31, information indicating that re-examination is necessary is added to the analysis result. As described above, in the present embodiment, quality control determination of the test samples (S1001 to 1006) included on one sample set (sample set A in FIG. 10) and test samples (S1039 to 1046) included in another sample set (sample set B in FIG. 10) is performed based on the measurement results of the control sample contained in one sample set (sample set A in FIG. 10).

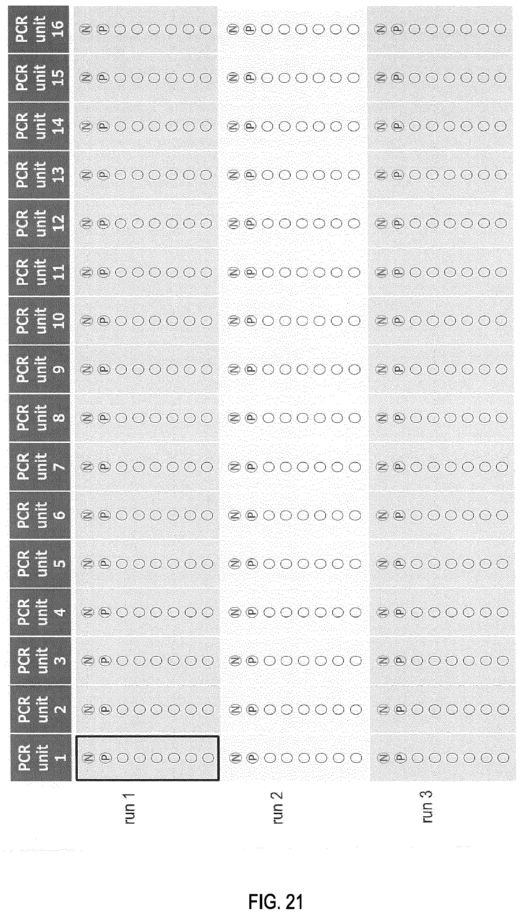

According to the present embodiment, the number of measurements of the control sample can be significantly reduced compared with the case in which the accuracy is controlled based on the conventional concept (see FIG. 21 described later), such that the reagent cost can be reduced and the throughput can be improved. Even if the number of measurements of the control sample is reduced, at least one positive control sample and one negative control sample are measured in the same QC group using a common mixed reagent, so that the accuracy of the reagent can be guaranteed. More specifically, if the measurement results of the positive control sample are valid, it is confirmed that the mixed reagent is properly prepared and the nucleic acid is properly amplified by the enzymes and primers contained in the mixed reagent. If the measurement result of the negative control sample is valid, it can be confirmed that at least the mixed reagent has no contamination. That is, according to the nucleic acid analysis method of the present embodiment, the quality of the mixed reagent, specifically, the suitability of preparation and the presence or absence of contamination is guaranteed while reducing the reagent cost and improving the throughput for measuring the control sample, and at the same time, nucleic acid analysis of the test sample becomes possible.

The mixed reagent used for sample preparation has a great influence on the analysis accuracy of the inspection system provided with the nucleic acid analyzer 1. That is, there is a problem in the preparation of the mixed reagent or the enzyme reagent/primer reagent itself which is the raw material, and if the mixed reagent of the desired quality is not prepared, accurate measurement data cannot be obtained. Since it is highly possible that mixed reagent of the target quality was not prepared if it is determined that there is an abnormality in the quality control determination, in this embodiment, it is assumed that the analysis accuracy is abnormal for all the samples in the same QC group. Note that although the measurement accuracy derived from the equipment of the PCR unit 21 is guaranteed by various sensors installed in the unit, it is for example, appropriate that the control sample should be measured periodically as shown in the modification described later.

Note that the measurement may be stopped if the PCR control unit 33 has not completed the measurement of the test samples of the same QC group in the different PCR units U2 to U6 when the quality control determination of the abnormality is performed. In this case, unnecessary measurement can be eliminated, which leads to improvement in throughput. In the example shown in FIG. 9, when the quality control determination of abnormality is made and the character "NG" is displayed in the analysis result column of the test sample of the same sample group, the data analysis of the test sample is not performed; however, data analysis also may be performed on the test sample displayed as NG, and the Ct value may be calculated.

In the example of FIG. 10, the DB 1000 includes only the Ct value of the test sample in the analysis result record, but as a result of comparing the Ct value of the test sample with a predetermined threshold value, a positive/negative determination result for each sample may be stored. If the Ct value is less than the threshold value, it can be determined to be positive, and if the Ct value is greater than or equal to the threshold value, it can be determined to be negative. Note that the result of the quality control determination stored in column C6 of FIG. 10 is not limited to OK/NG insofar as it is sufficient that the presence or absence of the quality control abnormality can be distinguished. For example, it may be "o/x", "pass / fail" and the like, or "0" may indicate that there is no quality control abnormality, and "1" may indicate that there is an abnormality.

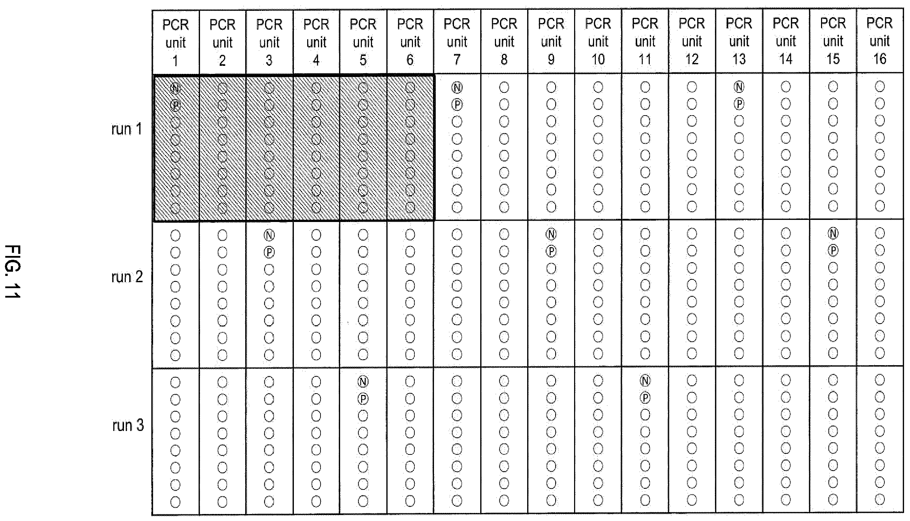

FIG. 11 is a diagram showing an example of sample arrangement in the PCR unit group 20 (hereinafter referred to as "Example 1"). In FIG. 11, "P" means a positive control sample, "N" means a negative control sample, and an uncharacterized ○ means a test sample.

In Example 1, a positive control sample and a negative control sample are measured for each mixed reagent prepared at one time, and the measurement results of the control sample are applied to the measurement results of a plurality of sample sets measured by a PCR unit 21 for measuring the control sample. As described above, in the sample preparation unit, the enzyme reagent and the primer reagent are mixed to prepare a plurality of (for example, 48 tests) of mixed reagents in the mixed reagent container 16. At least one sample set containing the control sample is prepared for each new preparation of the mixing reagents. In Example 1, 48 test samples are prepared using the same mixed reagents. In other words, six sample sets containing 8 samples are produced.

The positive control sample and the negative control sample are included in the first sample set prepared first among the six sample sets included in the same QC group (hatched portion in FIG. 11) prepared using the same mixed reagent. The first sample set is a sample set among the six sample sets that is first transported to the PCR unit 21 and the measurement is started. In this case, since the quality control determination based on the measurement data of the control sample is performed first, the analysis result of the test sample (subject sample) can be returned as soon as the measurement of the first sample set is completed, and, hence, the turnaround time (TAT) from the exam request to the result report can be shortened.

In Example 1, six sample sets of the first QC group prepared from the same mixed reagent are measured by PCR units U1 to U6, and six sample sets of the second QC group are measured by PCR units U7 to U12. The six sample sets of the third QC group are measured in PCR units U13-U16, and U1, U2. By the time the measurement of the sample set of the third QC group is started, the measurement of the sample set of the first QC group in the PCR units U1 and U2 is completed, and the 8-tube unit 40 has been removed, such that the sample set of the third QC group can be transported to PCR units U1 and U2.

Since up to eight sample can be measured in parallel using a plurality of PCR units 21, the wait time until sample collection can be shortened and the TAT of the earliest collected sample is also reduced compared to a conventional analyzer that batch processes a large number of samples using a well plate. Each sample set is sequentially distributed to a plurality of PCR units 21 according to a predetermined rule. In Example 1, the PCR units U1, U2, U3 ... U16, U1, U2 ... Are distributed in the order in which the sample sets are prepared. Efficient processing and measurement are possible by regularly distributing each sample set to a plurality of PCR units U1 to U16. In Example 1, the operation of the device is controlled so that the measurement of the first sample set of the first QC group was completed and the 8-tube unit 40 was removed by the time the fifth sample set of the third QC group was transferred to the PCR unit U1.

In Example 1, as described above, one positive control sample and one negative control sample are prepared for one QC group. The positive control sample and the negative control sample are contained only in the first sample set prepared in each QC group, and are not included in the other second to sixth sample sets. In Example 1, the first and second sample sets are made in a ratio of 1: 5. In this case, 46 out of 48 samples constituting one QC group are test samples.

FIG. 21 is a diagram showing a sample arrangement based on the conventional concept. Based on the conventional concept, as shown in FIG. 21, a sample set containing one positive control sample and one negative control sample is measured by each PCR unit. In this case, 1/4 of the total measurement is the measurement of the control sample. Therefore, if the conventional concept is applied to the nucleic acid analyzer 1, the throughput is lowered and the reagent cost is increased. On the other hand, according to the method of Example 1, 46 out of 48 measurements are the measurement of the subject sample, and the measurement of the control sample is reduced to 1/24 of the whole measurement. Therefore, according to the method of Example 1, a significant reduction in reagent cost and an improvement in throughput can be realized as compared with the method based on the conventional concept.

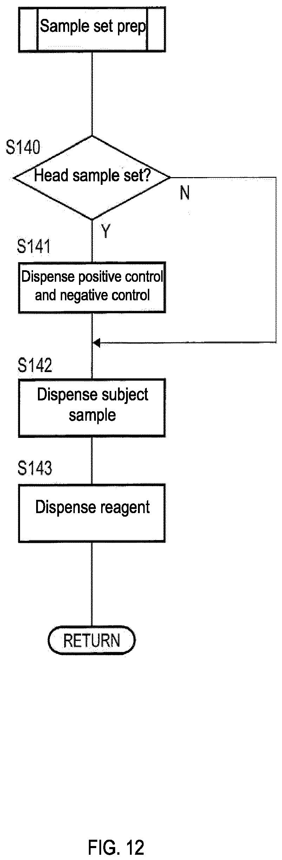

FIG. 12 is a flowchart showing a sample set preparation process corresponding to the first embodiment. In Example 1, as described above, six sample sets are prepared as one QC group prepared from the same mixed reagents. The six sample sets are produced by the sample preparation robot 11 under the control of the robot control unit 32. In step S140, the robot control unit 32 determines whether the sample set to be produced is the first sample set to be produced in the QC group. Hereinafter, the sample set produced first in the QC group is referred to as a head sample set. When the control sample set is the first sample set (YES in step S140), the sample preparation robot 11 respectively dispenses the positive control sample and the negative control samples into the container 41 of the 8-tube unit 40 under the control of the robot control unit 32.

Next, the sample preparation robot 11 dispenses the subject sample in the wells of the well plate 3 into the remaining six containers 41 of the 8-tube unit 40 (step S142). Next, the sample preparation robot 11 dispenses the mixed reagent into each container 41 of the 8-tube unit 40 to prepare a control sample and a test sample (step S143). Through the above process, a sample set containing two control samples and six test samples is prepared. Note that in the present embodiment, the test subject sample and the mixed reagent are dispensed into the 8-tube unit 40 in this order, but the order may be reversed.

In the determination of step S140, when the sample set to be prepared is not the first sample set, that is, when the second and subsequent sample sets in the QC group are prepared, the step of preparing the control sample in step S141 is skipped and the process advances to step S142. That is, the sample preparation robot 11 dispenses the subject sample into all the first to eighth containers 41 of the eight-tube unit 40 to prepare a sample set containing eight test samples.

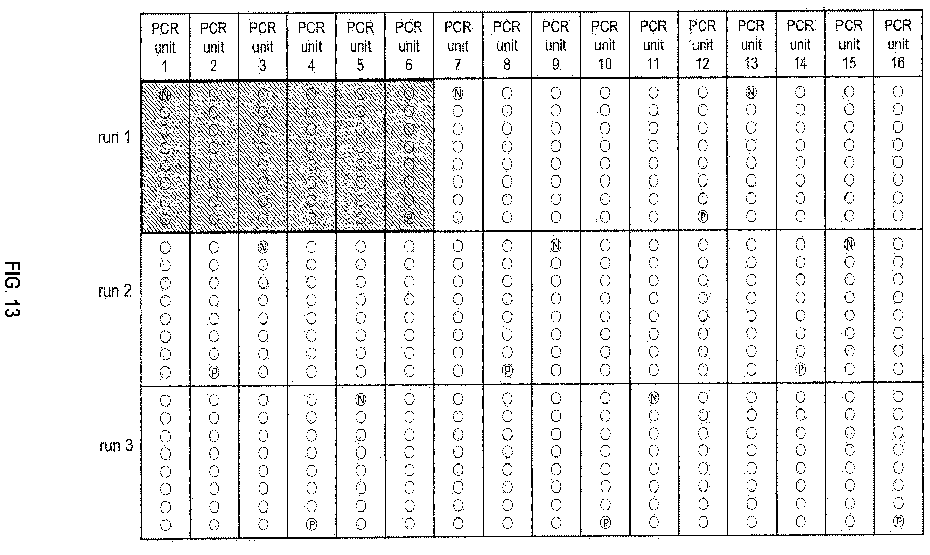

FIG. 13 is a diagram showing another example of sample arrangement in the PCR unit group 20 (hereinafter referred to as "Example 2"). The differences from the first embodiment will be described in detail below.

In Example 2, six sample sets of the same QC group contain one positive control sample and one negative control sample, and, in common with Example 1, applies the measurement results of the control samples measured by one PCR unit 21 to the measurement results of the plurality of sample sets measured by another PCR unit 21. On the other hand, Example 2 is different from Example 1 in that a positive control sample and a negative control sample are contained in different sample sets. In Example 2, of the six sample sets configuring the QC group, the first sample set produced contains a negative control sample, and the last sample set produced contains a positive control sample.

In Example 2, the first sample set containing the negative control sample, the second to fifth sample sets containing only the test sample, and the last sample set containing the positive control sample were prepared and transported to the PCR unit U1 to U6 in this order for measurement. In this case, the risk of contamination of the positive control with the negative control sample or the test sample can be reduced. The negative control sample is preferably prepared prior to the test sample in the first sample set and is housed in the first container 41 of the 8-tube unit 40. The positive control sample is preferably prepared after the test sample in the final sample set and is housed in the eighth container 41 of the 8-tube unit 40.

In Example 2, a sample set containing a control sample (a first sample set and a last sample set) and a sample set containing only a test sample are prepared at a ratio of 1: 2. However, since the first sample set and the last sample set contain one control sample, 46 of the 48 test samples configuring one QC group are also test samples.

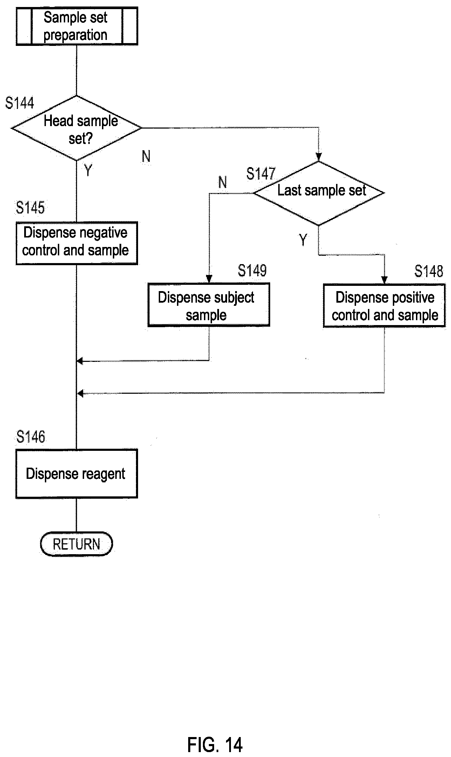

FIG. 14 is a flowchart showing a process for preparing the sample set of Example 2. In step S144, the robot control unit 32 determines whether the sample set to be produced is the head sample set among the six sample sets configuring the same QC group (step S144). In the case of the first sample set (YES in step S144), the robot control unit 32 controls the sample preparation robot 11 so as to dispense the negative control and the subject sample into each container 41 of the 8-tube unit 40 (stepS145). In step S145, the sample preparation robot 11 dispenses the negative control sample into the first container 41 of the 8-tube unit 40 under the control of the robot control unit 32, and subsequently, dispenses the subject sample from of the well plate 3 into the second to eighth containers 41 of the 8-tube unit 40. Next, the sample preparation robot 11 dispenses the mixed reagent from the mixed reagent container 16 into each container 41 of the 8-tube unit 40 to prepare a negative control sample and a test sample (step S146).

If the determination is NO in step S144, then the robot control unit 32 determines whether the sample set to be produced is the last sample set among the six sample sets configuring the same QC group (step S147). When the robot control unit 32 determines that it is the last sample set (YES in step S147), the robot control unit 32 controls the sample preparation robot 11 so as to dispense the positive control sample and the subject sample into each container 41 of the 8-tube unit 40 (step S148). In step S148, the subject sample is dispensed into the 1st to 7th containers 41 of the 8-tube unit 40, and then the positive sample is dispensed into the 8th container 41.

When the sample set to be prepared is neither the first sample set nor the last sample set, that is, when the second to fifth sample sets in the QC group are prepared, the subject sample is dispensed into each container 41 of the 8-tube unit 40 (step S149). When steps S148 and 149 are completed, the process proceeds to the step of dispensing the mixed reagent in step S146.

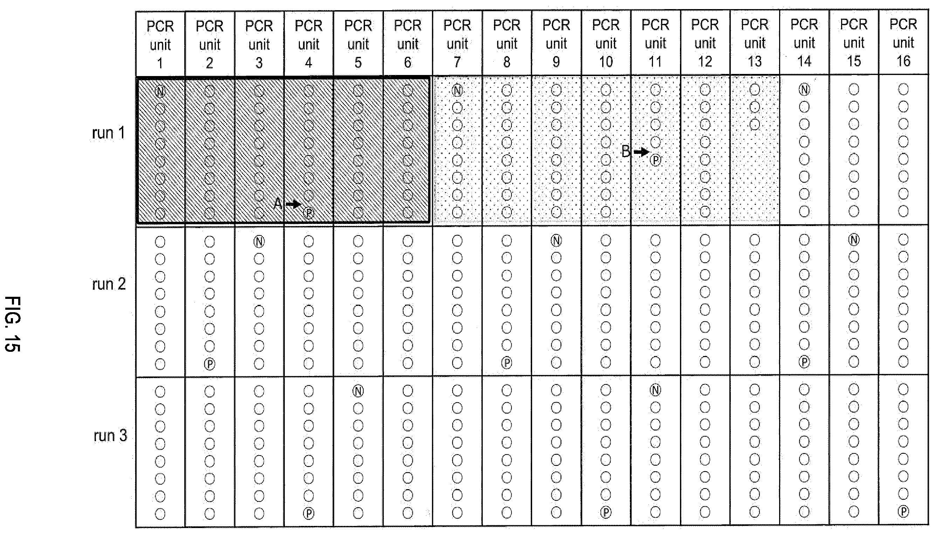

FIG. 15 is a diagram showing another example of sample arrangement in the PCR unit group 20 (hereinafter referred to as "Example 3").

Example 3 has an aspect in common with Example 2 in that a plurality of sample sets configuring the same QC group contain one positive control sample and one negative control sample, and the positive control sample and the negative control sample are contained in different sample sets. In Example 2, a positive control sample was included in the sample set prepared last among the sample sets of the same QC group. In other words, the final sample set that can be made with one mixed reagent includes a positive control sample. On the other hand, the third embodiment considers the case in which the test request is interrupted and time elapses until the next test subject sample is supplied. In Example 3, even if a mixed reagent remains and is capable of being used to prepare a number of samples in excess of the maximum number of samples (8) contained in the sample set, the sample set containing the positive control sample is prepared when the test request is interrupted.

In FIG. 15, arrows A and B indicate the time when the test request is interrupted, that is, the time when the supply of the well plate 3 containing the subject sample is temporarily stopped. At the time of arrow A, the sample preparation unit is preparing the fourth sample set that configures the same QC group, and the mixed reagents for 8 tests or more remain, but the positive control sample is included in the fourth sample set. Similarly, at the time point of arrow B, the mixed reagent for 8 tests or more remains, but the positive control sample is included in the 5th sample set. When the well plate 3 containing the subject sample is continuously supplied to the nucleic acid analyzer 1 without interruption, a positive control sample is prepared in the last sample set of the same QC group as in Example 2.

In this way, when the supply of the test subject sample is interrupted, the positive control sample is included in the sample set even if the sample set being prepared is not the last sample set in the same QC group. If the supply of the subject sample is interrupted and time is required to prepare the final sample set, the positive control sample can be measured first, and the measurement result can be quickly analyzed for the test sample for which the measurement has been completed. According to Example 3, when the supply of the subject sample to the nucleic acid analyzer 1 is intermittent, the TAT can be significantly shortened as compared with Example 2.

In Example 3, when the supply of the subject sample is resumed, a sample set is prepared using the remaining mixed reagents. If a sample set with a vacancy in a container 41 of the 8-tube unit 40 is prepared when the supply of the subject sample is interrupted, seven or more sample sets are prepared for one QC group. Note that since the positive control sample has already been prepared, it is not necessary to prepare the positive control sample in the final sample set of the same QC group. If the measurement of the positive control sample is completed, the analysis result of the test sample can be returned as soon as the measurement of the remaining sample set is completed.

In Example 3, the following four types of sample sets can be prepared as sample sets of the same QC group. In Example 3, the sample set of the following type (2) is prepared only when the supply of the subject sample is interrupted during the preparation of the sample set.
(1) Sample set including negative control sample and test sample
(2) A sample set containing a negative control sample, a positive control sample, and a test sample.
(3) Sample set including positive control sample and test sample
(4) Sample set containing only inspection samples

In the sample sets of the same QC group, the sample set of (1) above is the sample set to be prepared first, and it is produced when the number of subject samples supplied is greater than the number of containers 41 of the 8-tube unit 40 containing the sample set. The sample set of (2) above is the first sample set, and is prepared when one or more containers 41 of the 8-tube unit 40 are left over after dispensing the negative control and all the supplied subject samples. The sample set of (3) above is the second and subsequent sample sets, and the positive control is undispensed and the remaining amount of the mixed reagent is one sample, or the positive control is undispensed; it is produced when one or more containers 41 of the 8-tube unit 40 are left over after dispensing of all the supplied subject samples. The sample set of (4) above is prepared when the preparation conditions of the sample sets of (1) to (3) are not satisfied.

According to Examples 1 to 3, quality control of test samples belonging to the same QC group is performed based on one positive control sample and one negative control sample being prepared for one QC group, and the measurement results of the positive control sample and the negative control sample. If there is no problem with the measurement results of the positive control sample and the negative control sample, it can be confirmed that there is no abnormality due to improper preparation of the mixed reagent or contamination for test samples of the same QC group prepared using the same mixed reagent. Therefore, according to Example 1, test sample nucleic acid analysis is possible while confirming the presence or absence of an abnormality due to poor preparation of the mixed reagent or contamination based on the positive control sample and the negative control sample included in one QC group, the nucleic acid of the test sample is confirmed.

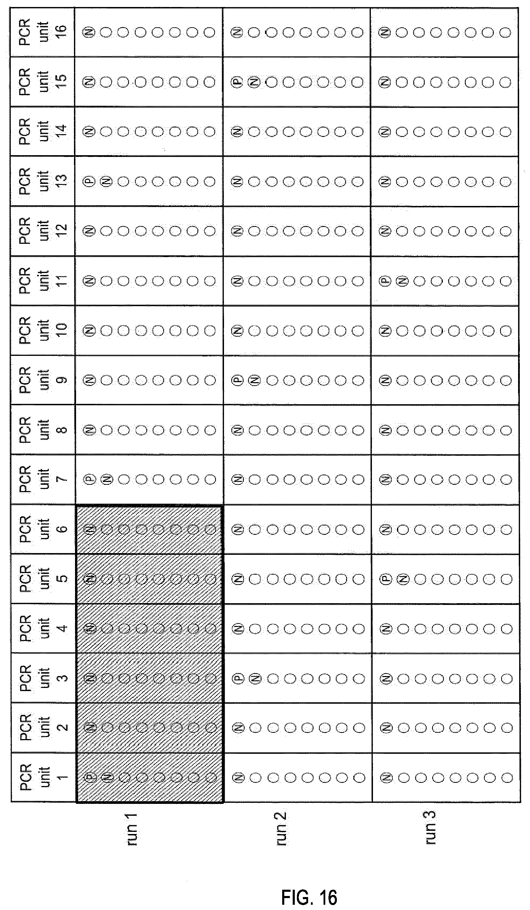

FIG. 16 is a diagram showing another example of sample arrangement in the PCR unit group 20 (hereinafter referred to as "Example 4").

Example 4 is common to Example 1 in that, of the six sample sets of the same QC group, the sample set prepared first contains one positive control sample and one negative control sample. On the other hand, Example 4 is different from Example 1 in that a negative control sample is also contained in a sample set other than the first sample set. In the first sample set of Example 4, the positive control sample is contained in the first container 41 of the 8-tube unit 40, and the negative control sample is contained in the second container 41; the order of the control samples may be reversed.

In Example 4, in the quality control based on the positive control sample, the measurement result of the positive control sample included in the first sample set is applied to the quality control determination of the test sample of the same QC group. For the negative control sample, on the other hand, the quality control determination of the test sample included in each sample set is performed based on the measurement result of the negative control sample included in each sample set. For example, in the example of FIG. 16, the sample set A measured by Run1 of PCR unit 1 contains a positive control sample and a negative control sample, and the sample set B measured by Run1 of PCR unit 2 contains a negative control sample. In this case, when the quality control determination results of the positive control sample and the negative control sample included in the sample set A are both OK, the accuracy control determination results of the six test samples included in the sample set A are also OK. For the test sample included in the sample set B, when the quality control determination of the positive control sample contained in the sample set A is OK and the quality control determination of the negative control sample contained in the sample set B is OK , the quality control determination result is OK.

According to Example 4, nucleic acid analysis is possible while confirming there is no abnormality due to poor preparation or contamination of the mixed reagents as in Examples 1 to 3. since one positive control sample and one negative control sample are contained in one QC group. In Example 4, since each sample set contains a negative control sample, contamination at the time of preparing each sample set can also be detected.

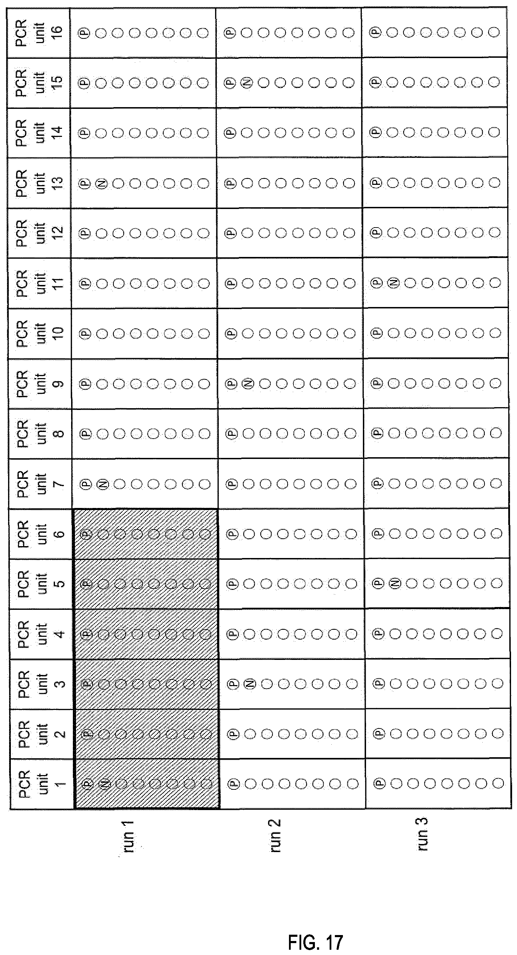

FIG. 17 is a diagram showing another example of sample arrangement in the PCR unit group 20 (hereinafter referred to as "Example 5").

Example 5 is common to Examples 1 and 4 in that, of the six sample sets of the same QC group, one positive control sample and one negative control sample are contained in the sample set prepared first. On the other hand, Example 5 is different from Examples 1 and 4 in that a positive control sample is also contained in another sample set other than the first sample set. In the first sample set of Example 5, the positive control sample is contained in the first container 41 of the 8-tube unit 40, and the negative control sample is contained in the second container 41; the order of the control samples also may be reversed. The concentration of the positive control in the positive control sample of the second sample set may be different from the concentration of the positive control sample of the first sample set; for example, the concentration of the detection sensitivity guaranteed by the PCR unit 21. Alternatively, the concentration may be ± 1 to 10% of the concentration of the detection sensitivity.

According to Example 5, nucleic acid analysis of the test sample is possible while confirming there is no abnormality due to poor preparation or contamination of the mixed reagents as in Examples 1 to 3 since one positive control sample and one negative control sample are contained in one QC group. In Example 5, since each sample set contains a positive control sample, it can be confirmed that each PCR unit 21 is operating normally, and nucleic acid amplification and detection of amplified nucleic acid are normally performed.

In Examples 4 and 5, in the second sample set, the control sample is contained in the first container 41 of the 8-tube unit 40, but may be contained in another container 41. The ratio of the first and second sample sets is 1: 5, but it may be 1: n (n ≧ 2) (the same applies to other examples). Although the control sample is included in all of the second sample set, a part of the second sample set also may include only the test sample.

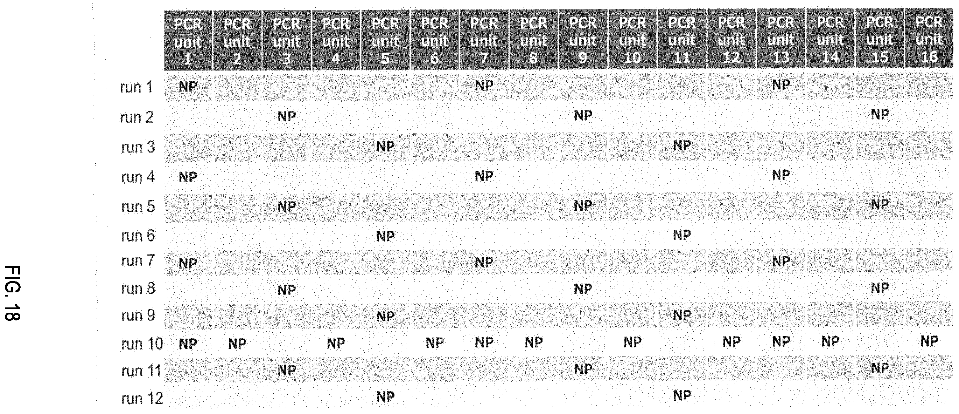

FIGS. 18 to 20 are views showing a modified example of the control sample arrangement in the PCR unit group 20. FIGS. 18 to 20 show only the arrangement of control samples of PCR units U1 to U16. "NP" means both a negative control sample and a positive control sample.

In the example shown in FIG. 18, control samples are periodically measured in a particular unit from the first measurement (run1) to the ninth measurement (run9) of each PCR unit U1 to U16. The arrangement of this control sample is the same as in the case of Example 1. Specifically, in the PCR units U1, U3, U5, U7, U9, U11, U13, U15, the control sample is measured once every three measurements. On the other hand, in the PCR units U2, U4, U6, U8, U10, U12, U14, U16, the control sample has not been measured even once until the ninth measurement. Therefore, when there is a unit among the PCR units U1 to U16 for which the control sample has not been measured within a predetermined time or a predetermined cycle, the control sample is automatically measured by the unit, and it is preferable the detection accuracy of the unit is periodically checked.

In the example shown in FIG. 18, the control sample is measured at the 10th measurement of the PCR units U2, U4, U6, U8, U10, U12, U14, U16. In this case, the control sample is always measured at least once every predetermined cycle (for example, once every 10 measurement cycles) in all the PCR units U1 to U16. For example, the robot control unit 32 counts the number of measurement cycles that do not include the measurement of the control sample for each of the PCR units U1 to U16, and when there is a unit in which the number of measurement cycles exceeds a predetermined threshold value, the robots 11 and 22 are controlled so as to transfer the sample set including the control sample to the unit, and the control sample is measured.

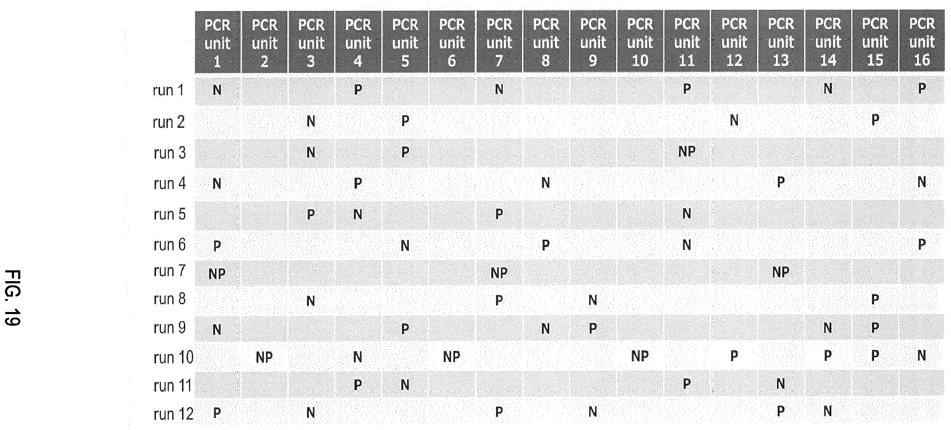

In the example shown in FIG. 19, the control sample is measured in all the PCR units U1 to U16, for example, once every 10 measurement cycles, as in the example shown in FIG. In the PCR units U2, U6, U10, the control sample was not measured even once until the 9th measurement, but when the above predetermined threshold value is set to 9 times, the control sample is measured during the tenth measurement by the of the PCR units U2, U6, U10. In the example shown in FIG. 19, a sample set containing only a positive control sample and a sample set containing only a negative control sample are transferred to a PCR unit as control samples, and a sample set containing both a positive control sample and a negative control sample is transported to the PCR unit.

Although a sample set containing a negative control sample is transported to and measured by the PCR units U12 and U14 by the 9th measurement, the measurement of the positive control sample is not done even once. In this case, in the 10th measurement of the PCR units U12 and U14, the sample set containing the positive control sample may be transported and and the positive control sample may be measured. The robot control unit 32 counts, for example, the number of measurement cycles not including the measurement of the positive control sample and the negative control sample for each PCR unit U1 to U16 and for each type of control sample. When there is a unit in which the number of measurement cycles that do not include the measurement of at least one control sample exceeds a predetermined threshold value, the robot control unit 32 controls the robots 11 and 22 to transport and measure the sample set containing the control sample that has not yet been measured.

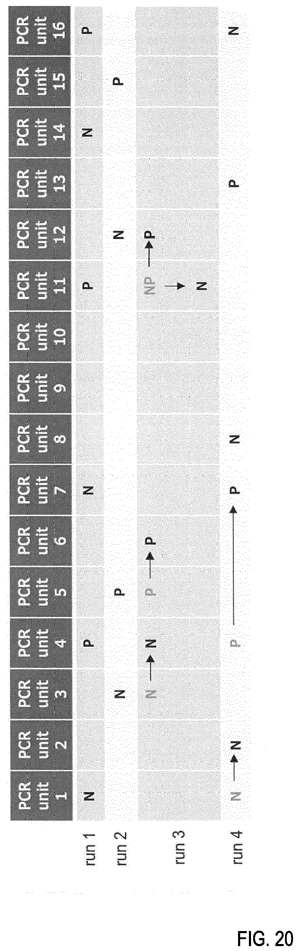

In the example shown in FIG. 20, when the measurement frequency of the control sample of the same type is high in the specific PCR unit 21, the PCR unit 21 for measuring the control sample is changed. That is, the order of the PCR units 21 (PCR unit 21 to which the sample set is distributed) is changed to disperse the destinations of the control samples. For example, in the PCR unit U3, the negative control sample is measured in the second measurement cycle, and the negative control sample is scheduled to be continuously measured in the third measurement cycle. As for the PCR unit U4, the positive control sample is measured in the first measurement cycle, and the positive control sample is scheduled to be measured again in the fourth measurement cycle, so that the measurement frequency of the positive control sample is high. In such a case, the PCR unit 21 for measuring the control sample is changed from the PCR unit 21 having a high measurement frequency of the control sample to a PCR unit 21 having a low measurement frequency of the control sample.

In the example shown in FIG. 20, a sample set containing a negative control sample that was to be distributed to the PCR unit U3 in the third measurement cycle is distributed to the PCR unit U4. A sample set containing a positive control sample that was to be distributed to the PCR unit U4 in the fourth measurement cycle is distributed to the PCR unit U7. Since the positive control samples have just been measured in the PCR units U5 and U6, this sample set is distributed to the PCR units U7. When a sample set containing two types of control samples is to be distributed as in the third measurement cycle of PCR unit U11, a sample set containing a positive control sample and a sample set containing a negative control sample also may be prepared instead of this sample set. Since the PCR unit U11 measures the positive control sample in the first measurement cycle, the sample set containing the positive control sample of the two sample sets is distributed to units other than the PCR unit U11.

According to the modification of FIGS. 18 to 20, it is possible to prevent the measurement frequency of the control sample from increasing in a specific PCR unit 21, and readily facilitate periodically checking the detection accuracy of each PCR unit 21. The robot control unit 32 counts, for example, the measurement frequency of the positive control sample and the negative control sample for each PCR unit U1 to U16 and for each type of control sample. Then, the measurement frequency of the control sample by a PCR unit exceeds a predetermined threshold value, the robots 11 and 22 are controlled so as to transfer the sample set including the control sample having a high measurement frequency to another unit. Another unit is a unit in which the measurement frequency of the control sample is less than or equal to a predetermined threshold value, and is determined, for example, based on a predetermined measurement order. Alternatively, a unit with a low measurement frequency of the control sample may be preferentially selected as another unit within a range that does not interfere with the overall measurement.

As described above, the nucleic acid analysis method of the above-described embodiments and modifications is a completely new and novel method in which the measurement result of the control sample measured by one PCR unit 21 is also used for the analysis of the test sample measured by another PCR unit 21. The measurement result of the control sample is applied, for example, to the analysis of a test sample prepared from the same reagent as the control sample, which is measured by another PCR unit 21. In this case, the number of measurements of the control sample can be significantly reduced. Therefore, the amount of the reagent used is reduced, the reagent cost is greatly reduced, and the ratio of the measurement of the test sample to the entire measurement is increased, so that the throughput is greatly improved. By appropriately setting the applicable range of the measurement result of the control sample, precise accuracy control can be performed even if the number of measurements of the control sample is reduced.

The present invention is not limited to the above-described embodiments and modifications, and the design can be appropriately changed insofar as the object of the present invention is not impaired. For example, although the configuration in which the positive control and the negative control are dispensed in the nucleic acid analyzer 1 to prepare the control reagent is exemplified, the positive control and the negative control also may be supplied from the upstream side of the test system as in the case of the subject sample.. The positive control and the negative control also may be supplied to the nucleic acid analyzer 1 by the belt conveyor 2 while being housed in the wells of the well plate 3.

Although the method of measuring at least one positive control sample and one negative control sample every time the mixed reagent prepared by mixing the enzyme reagent and the primer reagent changes in the above embodiment, positive and negative control samples also may be measured each time the production lot of reagents (for example, at least one of the enzyme and primer reagents) changes. For example, at least one sample set containing a positive control sample and a negative control sample is prepared each time the reagent production lot changes. In other words, if the production lots of the reagents that are the raw materials of the mixing reagents are the same even if the mixing reagents are changed, each sample prepared from the reagents of the same production lot is regarded as one QC group, and positive control samples and negative control samples may be measured at a rate of one per group.

Although eight samples housed in the eight-tube unit 40 are set as one sample set in the above embodiment, the form is not limited insofar as a plurality of samples housed in a plurality of connected containers are established as one sample set. For example, a maximum of 96 samples contained in a 96-well well plate also may be used as one sample set. In this case, in a preferred embodiment, the PCR unit may be configured to accept a 96-well well plate and perform at least nucleic acid amplification in batch processing on 96 samples. The PCR unit also may measure the amplified nucleic acid in addition to performing nucleic acid amplification.

Further, the reagent for preparing the sample may be single type. For example, when using an amount of reagent from a predetermined container to preparing a plurality of test samples, at least one sample set containing a positive control sample and a negative control sample is prepared each time the container is changed. When the positive control sample and the negative control sample are contained in different sample sets as in Example 2, at least one sample set containing a positive control sample and one sample set containing a negative control sample are prepared every time this container changes, or every time the reagent production lot changes.

In the above-described embodiment, an example is shown in which nucleic acid analysis is performed in parallel by a plurality of PCR units using a plurality of PCR units capable of independently amplifying nucleic acid and detecting amplified nucleic acid. Each PCR unit was equipped with a thermal cycler, a light source and a fluorescence detector. In another example of the invention, nucleic acid amplification and detection of amplified nucleic acid may be performed by different devices. For example, a plurality of thermal cycler modules capable of independently performing nucleic acid amplification may be used. In this case, the thermal cycler unit does not have to be provided with a light source and a fluorescence detector for detecting nucleic acid.

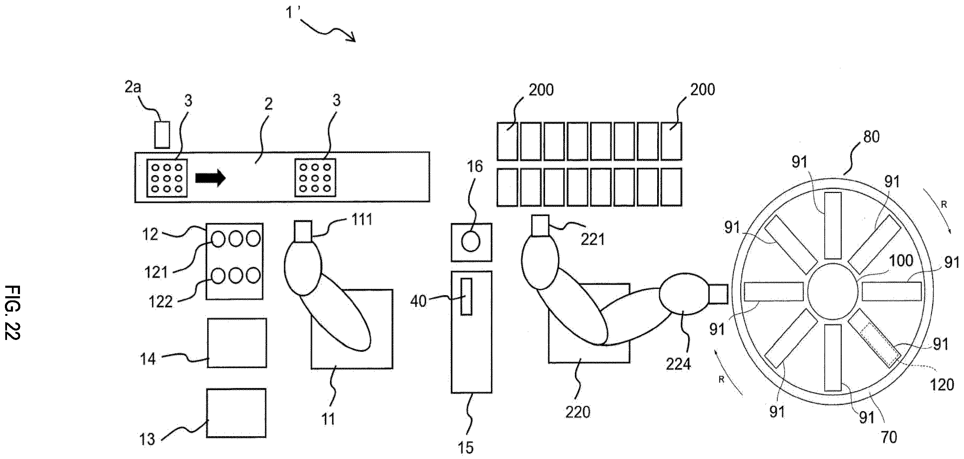

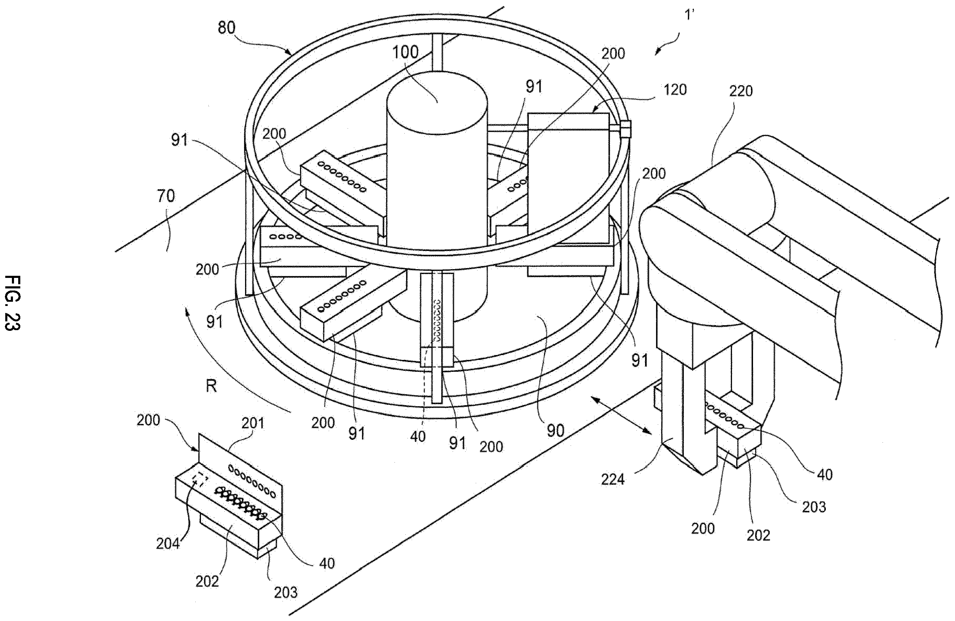

FIGS. 22 and 23 are schematic views showing other embodiments. In this example, the 8-tube unit 40 is set in the thermal cycler module 200 (hereinafter, simply referred to as the module 200).

FIG. 22 is a schematic diagram showing the overall configuration of the nucleic acid analyzer 1' according to a modified example. In FIG. 22, the same components as those in FIG. 1 are designated by the same reference numbers, and detailed description thereof will be omitted. In the modified example of FIG. 22, the measurement robot 220 includes an end effector 221 configured as a hand for placing the 8-tube unit 40 containing the sample in the module 200, and an end effector 224 configured by a hand for loading the module 200 accommodating the 8-tube unit 40 into a detection device 80.

As shown in FIG. 23, the detection device 80 is installed on, for example, a table 70. The detection device 80 has a disk-shaped rotary table 90 that can rotate around the central pillar 100, and a plurality of, for example, eight module mounting portions 91 arranged on the rotary table 90. Each module mounting portion 91 extends radially from the center of the rotary table 90. The module 200 can be mounted on each module mounting portion 91 with the longitudinal direction of the module 200 facing in the radial direction. The module mounting portions 91 are arranged at equal intervals in the circumferential direction R of the circle centered on the center of the rotary table 90. That is, the eight module mounting portions 91 are provided at intervals of 45 degrees in the circumferential direction R.

The detection device 80 includes an optical detector 120. The optical detector 120 has a shape extending radially outward from the center of the rotary table 90, and corresponds to one module mounting portion 91 (module 200 of the module mounting portion 91). The optical detector 120 detects fluorescence with nucleic acid amplification of a plurality of samples housed in a plurality of containers of a series of tubes 40 in the module 200 of the module mounting unit 91 when the module mounting unit 91 is positioned below the optical detector 120. That is, the optical detector 120 can detect fluorescence associated with nucleic acid amplification of a plurality of samples in module units, and is shared by eight modules 200.

The optical detector 120 has a light source unit and a photodetector unit. The optical detector 120 irradiates each container of the 8-tube unit 40 with light from the light source unit, and the fluorescence of the nucleic acid of the sample generated by the light can be detected by the optical detector unit. The light source unit includes a plurality of light emitting elements, for example, LEDs arranged in a row along the radial direction of the rotary table 90. Like the light source unit, the photodetector unit includes a plurality of light receiving elements, for example, photodiodes, which are arranged in a row along the radial direction of the rotary table 90. One light emitting element and one light receiving element are arranged in a pair, and the fluorescence generated by the light emitted by the light emitting element is detected by the paired light receiving elements.

The module 200 is provided inside the lid 201 provided with a plurality of holes corresponding to each container of the 8-tube unit 40, the main body 202 in which the 8-tube unit 40 is installed, the thermal cycler 203, and the main body 202. It is equipped with a CPU 204. The module 200 can be installed in the module mounting portion 91.

The measuring robot 223 grabs the module 200 by the arm 224 and sets it on the module mounting portion 91. When the module 200 is set in the module mounting portion 91, the rotary table 70 rotates. The CPU 204 controls the thermal cycler 203 with the module 200 set in the module mounting portion 91, and heats and cools the 8-tube unit 40 to perform nucleic acid amplification. The CPU is synchronized with the nucleic acid analyzer 1, and the cycle in which the module 200 goes around the rotary table 90 once is synchronized with one cycle of nucleic acid amplification consisting of heating and cooling. The nucleic acid analyzer 1 controls the rotary table 90 so that the module 200 is located directly under the fluorescence detector 120 at the fluorescence detection timing of each cycle of nucleic acid amplification. In this way one fluorescence detector 120 detects the nucleic acid amplified by the plurality of modules 200.

In this modification, nucleic acid amplification can be performed by the individual modules 200, and amplified nucleic acid can be detected using a common fluorescence detector 120. Also in this modification, the sample preparation robot 11 prepares a first sample set containing at least one control sample and a second sample set not containing at least one of the control samples contained in the first sample set. The prepared sample set can perform nucleic acid amplification and detection of amplified nucleic acid by the module 200 and the detection device 80.

The present disclosure includes following items:
Item 1: A nucleic acid analysis method comprising:
   preparing a first sample set including a test sample prepared from a first subject sample and a reagent, and a control sample prepared from at least one of a positive control and a negative control and the reagent;
   preparing a second sample set including a test sample prepared from a second subject sample and the reagent, and does not contain at least one of the control samples included in the first sample set;
   amplifying nucleic acid of the first sample set, and measuring the amplified nucleic acid;
   amplifying nucleic acid of the second sample set, and measuring the amplified nucleic acid; and
   analyzing the measurement results of each of the test samples included in the first and second sample sets based on at least the measurement result of the control sample included in the first sample set.
Item 2: The nucleic acid analysis method according to item 1, wherein
   the nucleic acid on the first sample set is amplified by a first unit that receives and amplifies one sample set; and
   the nucleic acid on the second sample set is amplified by a second unit that receives and amplifies the nucleic acid of one sample set.
Item 3: The nucleic acid analysis method according to item 2, wherein
   the nucleic acid amplification by the first unit and the nucleic acid amplification by the second unit are performed in parallel.
Item 4: The nucleic acid analysis method according to item 2 or 3, wherein
   the first and second units each include a detector that detects the amplified nucleic acid;
   the measurement of the amplified nucleic acid of the first sample set is performed by the detector of the first unit;
   the measurement of the amplified nucleic acid of the second sample set is performed by the detector of the second unit.
Item 5: The nucleic acid analysis method according to item 4, wherein
   the measurement of the amplified nucleic acid by the first unit and the measurement of the amplified nucleic acid by the second unit are performed in parallel.
Item 6: The nucleic acid analysis method according to any one of items 1 to 5, wherein
   the first and second sample sets are a plurality of samples accommodated in a plurality of connected containers.
Item 7: The nucleic acid analysis method according to any one of items 1 to 6, wherein
   the first sample set includes at least one control sample and a plurality of test samples.
Item 8: The nucleic acid analysis method according to any one of items 1 to 7, wherein
   the test sample included in the first sample set and the test sample included in the second sample set are a plurality of samples prepared from a plurality of subject samples, respectively.
Item 9: The nucleic acid analysis method according to any one of items 1 to 8, wherein
   the first sample set comprises a positive control sample prepared from a reagent and positive control, and a plurality of test samples.
Item 10: The nucleic acid analysis method according to item 9, wherein
   the first sample set further comprises a negative control prepared from a reagent and a negative control.
Item 11: The nucleic acid analysis method according to any one of items 1 to 10, wherein
   the first and second sample sets are prepared in a ratio of 1:n (n is an integer of 2 or more) using the same reagent.
Item 12: The method according to any one of items 1 to 11, wherein
   at least one set of the first sample set is prepared with respect to a plurality of sample sets prepared using a shared reagent.
Item 13: The nucleic acid analysis method according to any one of items 1 to 12, further comprising:
   preparing a mixed reagent as the reagent by mixing a first reagent and a second reagent;
   wherein at least one first sample set is prepared each time the mixed reagent is switched.
Item 14: The method according to any one of items 1 to 13, wherein
   at least one first sample set is prepared each time a production lot of the reagent to be used is switched.
Item 15: The nucleic acid analysis method according to any one of items 1 to 14, further comprising:
   preparing a third sample set including a test sample prepared from a third subject sample and the reagent, and a negative control sample prepared from a negative control and the reagent;
   amplifying the nucleic acid of the third sample set and measuring the nucleic acid;
   analyzing the measurement result of each test sample included in the first to third sample sets based on the measurement result of the positive control sample included in the first sample set and the measurement result of the negative control sample included in the third sample set.
Item 16: The nucleic acid analysis method according to items 1 to 15, wherein
   the second sample set includes a negative control sample prepared from a negative control;
   further comprising:
      analyzing the measurement result of the test sample included in the second sample set based on the measurement result of the positive control sample included in the first sample set and the measurement result of the negative control sample included in the second sample set.
Item 17: The nucleic acid analysis method according to items 1 to 17, wherein
   the second sample set includes a negative control sample prepared from a negative control;
   further comprising:
      analyzing the measurement result of the test sample included in the second sample set based on the measurement result of the negative control sample included in the first sample set and the measurement result of the positive control sample included in the second sample set.
Item 18: The nucleic acid analysis method according to any one of items 1 to 17, further comprising:
   distributing the plurality of the first sample sets and the plurality of the second sample sets to a plurality of units including the first to third units capable of individually amplifying nucleic acids with respect to the sample set;
   amplifying the nucleic acid distributed to the plurality of units of each sample set; and measuring the amplified nucleic acid.
Item 19: The nucleic acid analysis method according to item 18, wherein
   the plurality of first sample sets and the plurality of second sample sets are sequentially distributed to the plurality of units according to a predetermined rule.
Item 20: The nucleic acid analysis method according to item 18, wherein
   the first sample set is distributed to the units satisfying a predetermined condition among the plurality of units.
Item 21: The nucleic acid analysis method according to item 20, wherein
   the predetermined condition is that the control sample is not measured within a predetermined time or a predetermined cycle.
Item 22: A nucleic acid analyzer comprising:
   a sample preparation device that prepares a plurality of sample sets including a test sample prepared from a subject sample and a reagent;
   at least one unit that amplifies nucleic acid of each of the plurality of sample sets and measures the amplified nucleic acid; and
   a control unit;
   wherein the sample preparation device, under the control of the control unit, prepares a first sample set including a test sample, a control sample prepared from at least one of a positive control and a negative control, and a second sample set containing a test sample, and does not include at least one of the control samples included in the first sample set; and
   wherein the control unit analyzes the measurement results of each of the test samples included in the first sample set and second sample set based on at least the measurement results of the control sample included in the first sample set.

### EXPLANATION OF REFERENCE NUMBERS

- 1: Nucleic acid analyzer
- 2: Belt conveyor
- 3: well plate
- 11: Sample preparation robot
- 12: Reagent storage unit
- 13: QC sample storage unit
- 14: Nozzle tip storage unit
- 15: 8-tube storage unit
- 16: Mixing reagent container
- 20: PCR unit group
- 21,: U1-U16 PCR unit
- 22: Measurement robot
- 23: Thermal cycler
- 24: Tube holder
- 25: Cover
- 26: Optical unit
- 261: Light source
- 262: Fluorescence detector
- 27: Control unit
- 31: System control unit
- 311: CPU
- 312: Storage unit
- 313: Communication interface
- 314: Display unit
- 315: Input unit
- 32: Robot control unit
- 321: CPU
- 322: Storage unit
- 323: Communication interface
- 33: PCR control unit
- 331: CPU
- 332: Storage unit
- 333: Communication interface
- 40: 8-tube unit
- 41: Container
- 411: Container body
- 412: Lid
- 50: Host computer

## Claims

1. A nucleic acid analysis method comprising:
preparing a first sample set including a test sample prepared from a first subject sample and a reagent, and a control sample prepared from at least one of a positive control and a negative control and the reagent;
preparing a second sample set including a test sample prepared from a second subject sample and the reagent, and does not contain at least one of the control samples included in the first sample set;
amplifying nucleic acid of the first sample set, and measuring the amplified nucleic acid;
amplifying nucleic acid of the second sample set, and measuring the amplified nucleic acid; and
analyzing the measurement results of each of the test samples included in the first and second sample sets based on at least the measurement result of the control sample included in the first sample set.

2. The nucleic acid analysis method according to claim 1, wherein
the nucleic acid on the first sample set is amplified by a first unit that receives and amplifies one sample set; and
the nucleic acid on the second sample set is amplified by a second unit that receives and amplifies the nucleic acid of one sample set.

3. The nucleic acid analysis method according to claim 2, wherein
the nucleic acid amplification by the first unit and the nucleic acid amplification by the second unit are performed in parallel.

4. The nucleic acid analysis method according to claim 2 or 3, wherein
the first and second units each include a detector that detects the amplified nucleic acid;
the measurement of the amplified nucleic acid of the first sample set is performed by the detector of the first unit;
the measurement of the amplified nucleic acid of the second sample set is performed by the detector of the second unit.

5. The nucleic acid analysis method according to claim 4, wherein
the measurement of the amplified nucleic acid by the first unit and the measurement of the amplified nucleic acid by the second unit are performed in parallel.

6. The nucleic acid analysis method according to any one of claims 1 to 5, wherein
the first and second sample sets are a plurality of samples accommodated in a plurality of connected containers.

7. The nucleic acid analysis method according to any one of claims 1 to 6, wherein
the first sample set includes at least one control sample and a plurality of test samples.

8. The nucleic acid analysis method according to any one of claims 1 to 7, wherein
the test sample included in the first sample set and the test sample included in the second sample set are a plurality of samples prepared from a plurality of subject samples, respectively.

9. The nucleic acid analysis method according to any one of claims 1 to 8, wherein
the first sample set comprises a positive control sample prepared from a reagent and positive control, and a plurality of test samples.

10. The nucleic acid analysis method according to claim 9, wherein
the first sample set further comprises a negative control prepared from a reagent and a negative control.

11. The nucleic acid analysis method according to any one of claims 1 to 10, wherein
the first and second sample sets are prepared in a ratio of 1:n (n is an integer of 2 or more) using the same reagent.

12. The method according to any one of claims 1 to 11, wherein
at least one set of the first sample set is prepared with respect to a plurality of sample sets prepared using a shared reagent.

13. The nucleic acid analysis method according to any one of claims 1 to 12, further comprising:
preparing a mixed reagent as the reagent by mixing a first reagent and a second reagent;
wherein at least one first sample set is prepared each time the mixed reagent is switched.

14. The method according to any one of claims 1 to 13, wherein
at least one first sample set is prepared each time a production lot of the reagent to be used is switched.

15. A nucleic acid analyzer comprising:
a sample preparation device that prepares a plurality of sample sets including a test sample prepared from a subject sample and a reagent;
at least one unit that amplifies nucleic acid of each of the plurality of sample sets and measures the amplified nucleic acid; and
a control unit;
wherein the sample preparation device, under the control of the control unit, prepares a first sample set including a test sample, a control sample prepared from at least one of a positive control and a negative control, and a second sample set containing a test sample, and does not include at least one of the control samples included in the first sample set; and
wherein the control unit analyzes the measurement results of each of the test samples included in the first sample set and second sample set based on at least the measurement results of the control sample included in the first sample set.
